# EUROPÄISCHE PATENTSCHRIFT

(11) **EP 1 704 884 B1**
(45) Veröffentlichungstag und Bekanntmachung des Hinweises auf die Patenterteilung: **12.11.2008**
(21) Anmeldenummer: 05006543.2
(22) Anmeldetag: 24.03.2005
(51) Int. Cl.: A61M 5/142, A61M 5/145

(54) **Vorrichtung für die dosierte Verabreichung eines fluiden Produkts**
Device for the dosed administration of a fluid product
Dispositif pour la distribution dosée d'un produit fluide

(43) Veröffentlichungstag der Anmeldung: 27.09.2006
(73) Patentinhaber: Disetronic Licensing AG, 3401 Burgdorf (CH)
(72) Erfinder: Geiser, Simone, 4900 Langenthal (CH); Peter, Daniel, 3172 Niederwangen (CH)
(74) Vertreter: Wess, Wolfgang

(56) Entgegenhaltungen:
- EP-A- 0 514 816
- EP-A- 1 498 150
- WO-A-01/72357
- WO-A-20/04089448
- DE-A1- 19 717 107
- DE-C1- 4 133 402
- US-A- 5 492 534
- US-A1- 2004 122 366

## Beschreibung

Die Erfindung betrifft Vorrichtungen für die dosierte Verabreichung von fluiden Produkten in biotechnologischen Anwendungen, vorzugsweise in medizinischen einschließlich tiermedizinischen und pharmazeutischen Anwendungen. Insbesondere betrifft sie Infusionsgeräte und Injektionsgeräte.

In Therapien kommt der Genauigkeit der Dosierung der verabreichten Produkte eine große Bedeutung zu, beispielsweise der Verabreichung von Insulin in der Diabetestherapie. Gebräuchlich sind Infusionsgeräte und Injektionsgeräte, die ein zu verabreichendes Produkt mittels einer motorisch betriebenen Hubkolbenpumpe im Falle von Infusionsgeräten oder mittels einer manuell betätigten Hubkolbenpumpe im Falle von Injektionsgeräten aus einem Produktreservoir ausschütten. Bei Infusionsgeräten wird der Hubkolben üblicherweise von einem Rotationsantrieb angetrieben, wobei die Rotationsbewegung des Antriebs mittels eines Spindeltriebs in die Linearbewegung des Kolbens übertragen wird. Bei Injektionsgeräten dient ein Spindeltrieb häufig der Auswahl der zu verabreichenden Produktdosis, während die Linearbewegung des Kolbens unmittelbar manuell bewirkt wird. Bei Injektionsgeräten sind ferner Zahnstangengetriebe gebräuchlich. Den genannten Beispielen von Verabreichungsgeräten ist gemein, dass die Dosiergenauigkeit maßgeblich davon abhängt, wie genau die Weglänge vorgegeben werden kann, die der Kolben für die Förderung einer bestimmten Produktdosis zurücklegen muss.

Infusionsgeräte und Injektionsgeräte der vorstehend beschriebenen Art werden beispielsweise durch die DE 198 40 992 A, die DE 198 22 031 C und die DE 199 00 827 C beschrieben.

Besondere Anforderungen an die Dosiergenauigkeit und -feinheit stellen Infusionsgeräte, mit denen das Produkt oft über längere Zeiträume in kleinen, diskreten Boli abgegeben wird. Dabei kann es vorkommen, dass konstruktive Merkmale, die im Grunde der Verbesserung der Dosiergenauigkeit dienen, gleichzeitig auch einen störenden Einfluss ausüben können, so beispielsweise die Fähigkeit der Okklusionserkennung. Ein Infusionsgerät mit einer vorteilhaft gestalteten, automatischen Okklusionserkennung wird in der DE 198 40 992 beschrieben, die diesbezüglich hiermit auch für die Zwecke der Erfindung in Bezug genommen wird. Ein weiteres Gerät mit Okklusionserkennung beschreibt die WO 01/72357 A2. Für die Okklusionserkennung wird die Fördereinrichtung im Ganzen über einen Sensor an dem Gehäuse des Infusionsgeräts abgestützt. Um zu verhindern, dass durch diese Art der Abstützung Relativbewegungen zwischen der Fördereinrichtung und dem Produktbehältnis ermöglicht werden, schlägt die WO 01/72357 A2 für den Zusammenbau des Geräts vor, die gesamte Fördereinrichtung in Förderrichtung des Kolbens zunächst bis gegen einen von dem Gehäuse gebildeten Anschlag zu drücken, anschließend die Fördereinrichtung im Wesentlichen von dem Druck zu entlasten und schließlich eine Abschlusskappe in eine rückwärtige Gehäuseöffnung einzusetzen und mit dem Gehäuse zu verkleben. Die Kappe soll die Fördereinrichtung auf Anschlag gegen das Gehäuse halten. Als alternative Ausführung wird ferner vorgeschlagen, die Fördereinrichtung an ihrer von dem Kolben abgewandten Seite mittels eines elastischen Dichtrings an dem rückwärtigen Boden des Gehäuses abzustützen und einen zwischen der Rückseite der Fördereinrichtung und dem Gehäuseboden verbleibenden Hohlraum mit einem Füllmaterial auszugießen, beispielsweise mit Silikon. Das Füllmaterial soll im Wesentlichen inkompressibel sein, um den Sensor nicht zu entlasten.

Aus der DE 41 33 402 C1 ist ein Druckinfusionsgerät bekannt. Das Druckinfusionsgerät umfasst zwei Halter 11, 15 zur Aufnahmen einer Spritze 13, eine Spindel 20 als Antriebsglied und ein Abtriebsglied, dass aus einer Mutter 18, einem Rohr 16 und dem Halter 15 besteht. Mutter 18, Rohr 16 und Halter 15 sind fest miteinander verbunden und bewegen sich nur gemeinsam in axiale Richtung. Die Mutter 18 wird durch Umspritzen des Gewindeabschnitts der Spindel 20 mit Kunststoff hergestellt, so dass das Gewinde der Mutter 18 exakt komplementär zu dem Spindelgewinde 19 geformt ist.

Es ist eine Aufgabe der Erfindung, mit Vorrichtungen für die dosierte Verabreichung fluider Produkte die gewünschte Produktdosis genauer als bisher zu fördern.

Eine Vorrichtung für die dosierte Verabreichung eines fluiden Produkts, wie die Erfindung sie betrifft, umfasst ein Gehäuse, ein Reservoir für das Produkt und eine Fördereinrichtung. Das Gehäuse kann das Reservoir unmittelbar selbst bilden. Vorzugsweise bildet jedoch ein Behältnis, beispielsweise eine Ampulle, das Reservoir. Das Behältnis wird von dem

Gehäuse in definierter Lage gehalten. Vorzugsweise ist solch ein Behältnis in das Gehäuse eingesetzt. Das Behältnis kann, wie bereits heute bei Ampullen üblich, fertig konfektioniert sein, indem es mit einer definierten Produktmenge gefüllt ist und auch bereits ein das Behältnis rückwärtig abdichtender Kolben in dem Behältnis aufgenommen ist. Fertig konfektionierte Ampullen dieser Art sind für die Selbstverabreichung von Insulin in der Diabetestherapie üblich. Bei dem Produkt kann es sich um das bereits genannte Insulin, ein Wachstumshormon und grundsätzlich jedes andere medizinisch oder beispielsweise kosmetisch wirksame Produkt handeln. Die erfindungsgemäße Vorrichtung ist bevorzugt für die Selbstverabreichung konzipiert.

Die Fördereinrichtung umfasst wenigstens ein Antriebsglied und wenigstens ein Abtriebsglied. Das Antriebsglied wird vorzugsweise motorisch, falls die Vorrichtung ein Infusionsgerät ist, und vorzugsweise manuell, falls die Vorrichtung ein Injektionsgerät ist, angetrieben, so dass es eine Antriebsbewegung ausführt. Das Antriebsglied und das Abtriebsglied sind miteinander mechanisch so gekoppelt, dass die Antriebsbewegung des Antriebsglieds eine Abtriebsbewegung des Abtriebsglieds bewirkt. Die Abtriebsbewegung ist oder umfasst eine Axialbewegung, die von dem Antriebsglied axial abgestützt wird. Der Axialbewegung kann eine andere Bewegung oder können mehrere andere Bewegungen überlagert sein. Vorzugsweise ist die Abtriebsbewegung jedoch eine reine, lineare Axialbewegung. Die Kopplung zwischen dem Antriebsglied und dem Abtriebsglied ist ein Flankeneingriff, zu dessen Bildung das Antriebsglied und das Abtriebsglied je wenigstens eine Eingriffsflanke aufweisen. Vorzugsweise sind sowohl die wenigstens eine Eingriffsflanke des Antriebsglieds unmittelbar an dem Antriebsglied als auch die wenigstens eine Eingriffsflanke des Abtriebsglieds unmittelbar an dem Abtriebsglied geformt. Die Antriebsbewegung kann eine Axialbewegung sein, wie dies insbesondere der Fall sein kann, wenn die Vorrichtung ein Injektionsgerät ist. Bevorzugter ist die Antriebsbewegung jedoch eine Rotationsbewegung und in diesem Fall besonders bevorzugt um eine Achse, entlang der das Abtriebsglied die Abtriebsbewegung ausführt.

Das Abtriebsglied kann bei der Abtriebsbewegung unmittelbar auf das in dem Reservoir befindliche Produkt wirken, indem es beispielsweise selbst einen Hubkolben bildet oder mit einem Hubkolben permanent verbunden ist. Es kann aber auch nur lose gegen einen Hubkolben drücken. Möglich ist auch eine Ausführung, in der das Abtriebsglied erst über ein Übertragungsglied oder mehrere Übertragungsglieder auf ein unmittelbar auf das Produkt wirkendes Förderelement, beispielsweise einen Hubkolben, wirkt, wenn es die Abtriebsbewegung ausführt. So kann die Fördereinrichtung insbesondere teleskopierbar sein, wie dies in der DE 197 17 107 A beschrieben ist, die diesbezüglich in Bezug genommen wird. Je zwei benachbarte, im Flankeneingriff befindliche Teleskopstufen bilden in solch einer Ausbildung ein Antriebsglied und ein Abtriebsglied gemäß der Erfindung.

Flankeneingriffe, wie sie insbesondere von Gewindeeingriffen und Zahneingriffen bekannt sind, weisen fertigungsbedingt quer zu den Eingriffsflanken ein axiales Spiel auf, das der Dosiergenauigkeit abträglich sein kann. Ein axiales Spiel kann sich insbesondere bei Auftreten des sogenannten Syphoning-Effekts bemerkbar machen, d.h. wenn es im Behältnis aufgrund der Ausströmverhältnisse zu einer Saugsituation kommt.

Nach der Erfindung ist jedoch eine Spielreduziereinrichtung vorgesehen, die sowohl mit dem Antriebsglied als auch mit dem Abtriebsglied je in einem Einstelleingriff ist, in dem die Spielreduziereinrichtung relativ zu dem Abtriebsglied und dem Antriebsglied in solche eine Einstellposition verstellt und in der Einstellposition so gesichert ist, dass das dem Flankeneingriff inhärente Axialspiel gegenüber den bekannten, auf Flankeneingriff beruhenden Kopplungen reduziert oder vorzugsweise gänzlich eliminiert ist. Der Einstelleingriff mit einem der beiden Glieder aus Antriebsglied und Abtriebsglied entspricht dem Flankeneingriff zwischen dem Antriebsglied und dem Abtriebsglied. Der andere Einstelleingriff gibt die Verstellbewegung der Spielreduziereinrichtung entlang ihres Verstellwegs vor. Die in diesem Einstelleingriff verfügbare Verstellweglänge ist vorzugsweise so lang, dass die Spielreduziereinrichtung in ihrer Einstellposition nicht auf Anschlag mit dem betreffenden Glied ist, sondern noch innerhalb der in diesem Eingriff verfügbaren Verstellweglänge. Die beiden Einstelleingriffe können insbesondere auch von gleicher Art oder vollkommen gleich sein. Im letzteren Fall geben sie den Verlauf des Verstellwegs gemeinsam vor.

Der Einstelleingriff mit dem Antriebsglied ist vorzugsweise form- und kraftschlüssig, besonders bevorzugt ist der Einstelleingriff ein Gewindeeingriff. Bezüglich des Einstelleingriffs mit dem Abtriebsglied gilt vorzugsweise das Gleiche. Die Ausbildung beider Einstelleingriffe der Spielreduziereinrichtung als Gewindeeingriff bietet sich insbesondere dann an, wenn der Flankeneingriff zwischen dem Antriebsglied und dem Abtriebsglied ebenfalls eine Gewindeeingriff ist, wie die Erfindung es bevorzugt. Es ist jedoch beispielsweise auch möglich, den Einstelleingriff, der die Verstellbewegung vorgibt, als Eingriff eines Eingriffsglieds der Spielreduziereinrichtung in eine Führungsbahn rein formschlüssig zu gestalten und mittels einer Elastizitätskraft den anderen der beiden Einstelleingriffe form- und kraftschlüssig zu bilden. Der die Verstellbewegung vorgebende Einstelleingriff ist in bevorzugten Ausführungen kontinuierlich in dem Sinne, dass das Axialspiel zwischen dem Antriebsglied und dem Abtriebsglied von seiner fertigungsbedingten Ausgangsgröße bis vorzugsweise auf den Wert Null kontinuierlich im Einstelleingriff verringert werden kann, wie dies beispielsweise der bevorzugte Gewindeeingriff ermöglicht. Der Gewindeeingriff bietet ferner den Vorteil, dass die Spielreduziereinrichtung durch den Einstelleingriff selbst in jeder über den Verstellweg eingenommenen Position axial gestützt wird.

In bevorzugten Ausführungen ist die Spielreduziereinrichtung an einem der Glieder aus Antriebsglied und Abtriebsglied gegen Axialbewegungen relativ zu dem betreffenden Glied gesichert. Die Sicherung kann insbesondere so gestaltet sein, dass die Spielreduziereinrichtung die Antriebsbewegung mitmacht, wenn die Sicherung zwischen dem Antriebsglied und der Spielreduziereinrichtung besteht, und die Abtriebsbewegung mitmacht, wenn die Sicherung zwischen der Spielreduziereinrichtung und dem Abtriebsglied besteht.

Im beispielhaften Fall des Gewindeeingriffs kann die Sicherung der Spielreduziereinrichtung in dem Einstelleingriff in der Einstellposition allein durch Selbsthemmung bewirkt werden. Vorzugsweise ist die Spielreduziereinrichtung in ihrer Einstellposition in dem gesicherten Einstelleingriff jedoch stoffschlüssig gesichert. Dies gilt auch, falls es sich hierbei um einen Gewindeeingriff handelt. Die stoffschlüssige Sicherung erfolgt vorzugsweise in dem Einstelleingriff mit dem Antriebsglied. Eine Sicherung an dem Abtriebsglied wäre grundsätzlich jedoch in kinematischer Umkehr ebenfalls möglich. Anstatt die Sicherung allein in einem einzigen der beiden Einstelleingriffe zu realisieren, kann die Sicherung auch erst im Zusammenwirken der Spielreduziereinrichtung mit dem Antriebsglied und dem Abtriebsglied realisiert werden, in diesem Fall vorzugsweise dadurch, dass die Spielreduziereinrichtung sowohl an dem Antriebsglied als auch an dem Abtriebsglied elastisch abgestützt ist.

Es soll nicht ausgeschlossen werden, dass eines aus Antriebsglied und Abtriebsglied eine Zahnstange und das andere ein in die Zahnstange eingreifender Mitnehmer ist. Solche Zahnstangentriebe sind bekannt, beispielsweise von Injektionspens, so dass auf Details nicht eingegangen werden muss. Für solch einen Zahnstangentrieb kann eine Spielreduziereinrichtung mittels eines weiteren Mitnehmers gebildet werden, so dass zwei Mitnehmer in die gleiche Zahnreihe eingreifen. Durch Verstellung des axialen Abstands der beiden Mitnehmer kann auch bei einem derartigen Zahnstangentrieb das toleranzbedingt von Hause aus vorhandene Axialspiel reduziert werden.

Falls die Fördereinrichtung teleskopierbar ist, so wird vorteilhafterweise zwischen jedem Paar von im Flankeneingriff befindlichen Teleskopstufen eine erfindungsgemäße Spielreduziereinrichtung vorgesehen.

Die Spielreduziereinrichtung ist vorzugsweise eingliedrig als ein einziges Einstellglied gebildet, das in beiden Einstelleingriffen und entsprechend dem zu reduzierenden Axialspiel zumindest axial steif ist. Im Falle einer mehrgliedrigen Spielreduziereinrichtung sollte solch eine Spielreduziereinrichtung zumindest dann axial in sich steif sein, wenn sie in der Einstellposition gesichert ist. So könnte beispielsweise eine zweigliedrige Spielreduziereinrichtung ein erstes Einstellglied aufweisen, das in einem Einstelleingriff mit dem Antriebsglied ist, und ein zweites Einstellglied, das in einem Einstelleingriff mit dem Abtriebsglied ist. Die beiden Einstellglieder würden bis in die Einstellposition hinein axial relativ zueinander verstellt und müssten in der Einstellposition axial aneinander festgelegt werden oder sich selbsttätig aneinander festlegen, um die axiale Steifigkeit zu erhalten.

In bevorzugten Verabreichungsvorrichtungen ist das Antriebsglied ein Rotationsglied, das um eine Rotationsachse rotatorisch bewegbar gelagert ist. Das Abtriebsglied ist ein translatorisch in eine Translationsrichtung bewegbares Translationsglied. Eine rotatorische Antriebsbewegung des Antriebsglieds in eine Antriebsrichtung bewirkt eine translatorische Abtriebsbewegung des Abtriebsglieds in die Translationsrichtung. Handelt es sich bei der Verabreichungsvorrichtung um ein Infusionsgerät, so ist das rotatorisch bewegbare Antriebsglied vorzugsweise so abgestützt, dass es sich relativ zu dem Reservoir, vorzugsweise relativ zu dem Gehäuse, in und gegen die Translationsrichtung des Abtriebsglieds nicht bewegen kann. Da das Abtriebsglied bei seiner Translationsbewegung sich in dem Flankeneingriff an dem Antriebsglied abstützt, würde eine unerwünschte Translationsbewegung des Antriebsglieds bereits aufgrund des bei den bekannten Drehlagerungen unvermeidbaren Axialspiels seiner für die Rotationsbewegung erforderlichen Drehlagerung in Reaktion auf die Abtriebsbewegung des Abtriebsglieds stattfinden.

In bevorzugten Weiterbildungen ist für derartige Fördereinrichtungen deshalb eine weitere Spielreduziereinrichtung vorgesehen, um das der Drehlagerung des Antriebsglieds inhärente Axialspiel zu reduzieren und vorzugsweise zu eliminieren. Die Drehlagerung umfasst einen Lagerkörper, der das Antriebsglied um seine Rotationsachse drehbar lagert. Der Lagerkörper kann mehrteilig sein, wobei die mehreren Teile in und gegen die Translationsrichtung steif miteinander verbunden sind, vorzugsweise ist er jedoch einteilig.
Er kann ferner unmittelbar von dem Gehäuse der Vorrichtung gebildet werden. Er kann aber auch separat gebildet und in das Gehäuse eingesetzt sein. Ferner entspricht es bevorzugten Ausführungsfbrmen, wenn der Lagerkörper in der Ausbildung als separater Lagerkörper eine Lagerhülse ist, die das Antriebsglied zumindest in einem axialen Abschnitt umgibt. Um das Axialspiel der Drehlagerung zu reduzieren, sind wenigstens zwei axiale Stützflächen der Drehlagerung axial steif mit dem Lagerkörper und wenigstens zwei weitere axiale Stützflächen der Drehlagerung axial steif mit dem Antriebsglied verbunden. In axial steifer Verbindung können die Stützflächen entweder unmittelbar von dem Lagerkörper oder dem Antriebsglied gebildet werden, oder es wird die betreffende Stützfläche von einem separaten Körper gebildet, der dann jedoch axial steif, vorzugsweise vollkommen steif, mit entweder dem Lagerkörper oder dem Antriebsglied verbunden ist.

Vorzugsweise wird wenigstens eine der Stützflächen von der weiteren Spielreduziereinrichtung gebildet, die entweder mit dem Antriebsglied oder vorzugsweise dem Lagerkörper in einem Einstelleingriff in solch eine Einstellposition verstellt und in der Einstellposition an dem Lagerkörper oder dem Antriebsglied axial so gesichert ist, dass das Axialspiel der Drehlagerung reduziert und vorzugsweise eliminiert wird. Falls das Antriebsglied und das Abtriebsglied Stufen einer teleskopierbaren Fördereinrichtung sind, wie die DE 197 17 107 A sie beispielsweise beschreibt, so bildet die Drehlagerung die Drehlagerung der Eingangsstufe der Fördereinrichtung.

Die Erfindung wind in Anspruch 1 definent. Bevorzugte Ausgestaltungen der Erfindung werden in den Unteransprüchen beschrieben.

Ausführungsbeispiele der Erfindung werden nachfolgend anhand von Figuren erläutert.

Es zeigen:
- Figur 1: eine Verabreichungsvorrichtung eines Ausführungsbeispiels in einem Längsschnitt,
- Figur 2: ein Teil der Verabreichungsvorrichtung in einem anderen Längsschnitt,
- Figur 3: ein vergrößertes Detail der Figur 2,
- Figur 4: eine Spielreduziereinrichtung in einer alternativen Ausführung,
- Figur 5: ein Einstellglied der Spielreduziereinrichtung der Figur 4, zweite
- Figur 6: eine Verabreichungsvorrichtung micht gemäß der Erfindung in einem Längsschnitt,
- Figur 7: ein Detail X der Figur 6,
- Figur 8: einen Federring einer Spielreduziereinrichtung der zweiten Verabreichungsvorrichtung,
- Figur 9: Komponenten der zweiten Verabreichungsvorrichtung in einer Explosionsdarstellung und
- Figur 10: das Translationsglied der zweiten Verabreichungsvorrichtung.

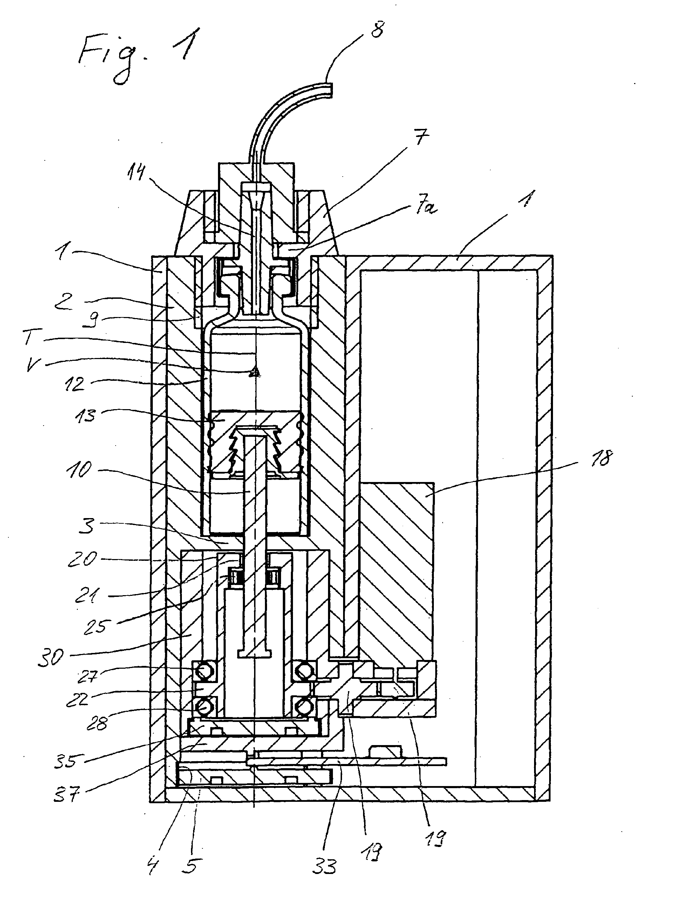

Figur 1 zeigt in einem Längsschnitt als Beispiel einer Verabreichungsvorrichtung ein Infusionsgerät. Das Gerät weist ein Gehäuse mit einer ersten Gehäusestruktur 1, und einer zweiten Gehäusestruktur 2, ein mit einem injizierbaren Produkt gefülltes Behältnis 12 und eine Fördereinrichtung auf, die das Produkt dosiert aus dem Behältnis 12 und durch einen sich anschließenden Katheter 8 fördert, um es zu verabreichen. Die Verabreichung kann insbesondere subkutan vorgenommen werden, wie dies beispielsweise in der Diabetestherapie üblich ist. Die erste Gehäusestruktur 1 umgibt die zweite Gehäusestruktur 2 und wird im folgenden als Hüllstruktur 1 bezeichnet. Die zweite Gehäusestruktur 2 stützt Komponenten des Infusionsgeräts und wird im folgenden als Stützstruktur 2 bezeichnet.

Das Behältnis 12 weist an einem vorderen Ende einen Auslass 14 auf, über den der Behältnisinnenraum mit dem Katheter 8 verbunden ist. In dem Behältnis 12 ist ein Kolben 13 entlang einer Translationsachse T in eine Förderrichtung V auf den Behältnisauslass 14 zu bewegbar aufgenommen. Das Behältnis 12 ist an seinem rückwärtigen Ende offen. Allerdings dichtet der Kolben 13 das Behältnis 12 zu dem rückwärtigen Ende hin ab.

Die Hüllstruktur 1 bildet eine feste Hülle, in der die Stützstruktur 2, die von einem Stützkörper 2 einstückig gebildet wird, eingesetzt und befestigt ist und ein Chassis des Gehäuses bildet. Die Hüllstruktur 1 und der Stützkörper 2 bilden das Gehäuse der Vorrichtung zumindest im Wesentlichen. Der Stützkörper 2 bildet einen ersten Aufnahmeraum, in dem das Behältnis 12 eingesetzt ist, und einen zweiten Aufnahmeraum für die Fördereinrichtung. Das Behältnis 12 ist mit seinem hinteren freien Rand auf Anschlag gegen einen, eine Stützschulter bildenden, nach radial einwärts ragenden Stützsteg 3 des Stützkörpers 2. Der von dem Stützkörper 2 gebildete erste Aufnahmeraum weist an dem vorderen Ende eine Öffnung auf, durch die das Behältnis 12 eingesetzt wird. Nach dem Einsetzen des Behältnisses 12 wird die Öffnung mit einem Deckel 7 verschlossen. Der Deckel 7 ist mit dem Stützkörper 2 verschraubt, der hierfür mit einem Gewinde 9 versehen ist. Der Stützkörper 2 und der Deckel 7 könnten auch mit je einer anderen Eingriffseinrichtung für den lösbaren Eingriff versehen sein, beispielsweise mit kooperierenden Rasteinrichtungen. Der Deckel 7 bildet ferner das Verbindungselement zwischen dem Katheter 8 und dem Auslass 14 des Behältnisses 12. Der Deckel 7 weist zu der Stützschulter des Stützstegs 3 eine Gegenstützschulter an einem Gegenstützsteg 7a auf, die gegen einen vorderen Rand des Behältnisses 12 und dadurch das Behältnis 12 auf Anschlag gegen den Stützsteg 3 drückt, so dass das Behältnis 12 relativ zu dem Stützkörper 2 axial festgelegt ist. Der Deckel 7 bildet somit ein vorderes Abschlusselement 7a, und der Stützsteg 3 bildet ein hinteres Abschlusselement 3 des ersten Aufnahmeraums. Der erste Aufnahmeraum ist ferner so geformt, dass das Behältnis 12 die korrekte Position und Ausrichtung in Bezug auf die Translationsachse T hat. Im Ergebnis stützt der Stützkörper 2 das Behältnis 12 axial in und gegen die Förderrichtung V formschlüssig, d.h. durch Kontakt mit den als Anschlägen dienenden Stegen 3 und 7a, ab. Die Fördereinrichtung wird in gleicher Weise axial zwischen anderen Anschlägen des Stützkörpers 2 abgestützt.

Die Fördereinrichtung umfasst den Kolben 13, ein Abtriebsglied 10, ein Antriebsglied 20 und einen motorischen Drehantrieb. Das Abtriebsglied 10 bildet ein Translationsglied der Fördereinrichtung, und das Antriebsglied 20 bildet ein Rotationsglied der Fördereinrichtung.

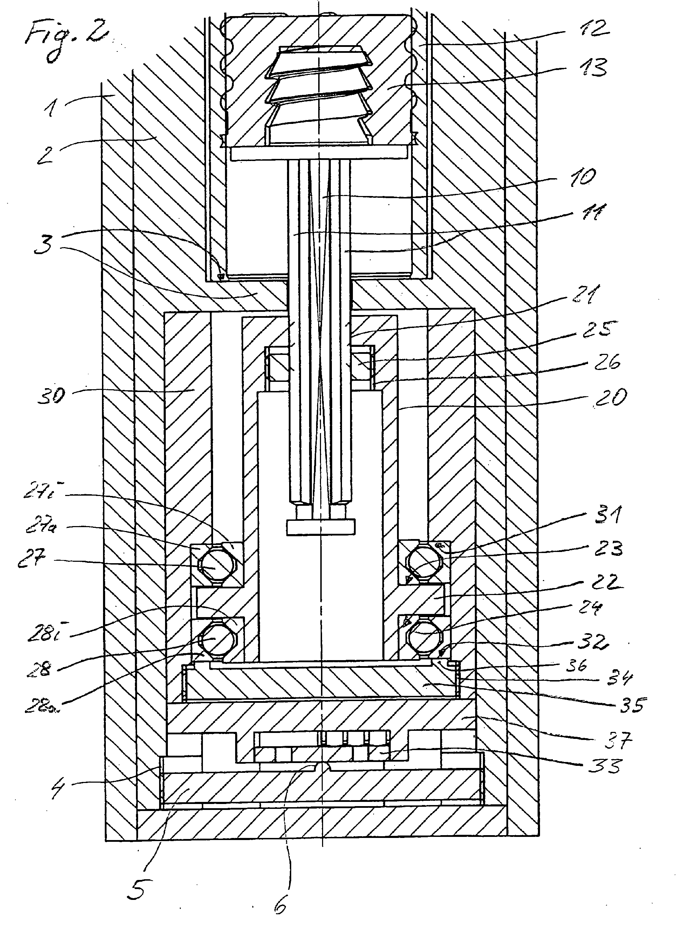

Das Abtriebsglied 10 ist eine Kolbengewindestange, d. h. eine Kolbenstange, die mit einem Gewinde versehen ist. Im Ausführungsbeispiel ist das Abtriebsglied 10 mit einem Außengewinde 11 versehen, das in dem Längsschnitt der Figur 2 erkennbar ist. Das Abtriebsglied 10 durchragt den Stützsteg 3, so dass es in den ersten Aufnahmeraum und den zweiten Aufnahmeraum des Stützkörpers 2 hinein ragt. An seinem vorderen Ende ist das Abtriebsglied 10 mit dem Kolben 13 verschraubt. Die Schraubverbindung wird mit dem Einsetzen des Behältnisses hergestellt. Der Stützsteg 3 führt das Abtriebsglied 10 linear entlang der Translationsachse T. Der Stützsteg 3 sichert das Abtriebsglied 10 so gegen eine Verdrehung relativ zu dem Stützkörper 2. Das Gewinde 11 wird im Ausführungsbeispiel hierfür von wenigstens einer axialen Nut oder Abplattung durchbrochen, in die der Stützsteg 3 eingreift.

Das Antriebsglied 20 ist gänzlich in dem zweiten Aufnahmeraum des Stützkörpers 2 angeordnet. Es ist rotationssymmetrisch zur Translationsachse T. Es ist hülsenförmig und kann daher auch als Antriebshülse bezeichnet werden. Das Antriebsglied 20 bildet an einem vorderen Hülsenende einen nach radial einwärts ragenden Steg, den das Abtriebsglied 10 durchragt. Das Antriebsglied 20 bildet an dem einwärts ragenden Steg ein Innengewinde 21, das mit dem Außengewinde 11 des Abtriebsglieds 10 in einem Gewindeeingriff ist.

Das Antriebsglied 20 ist um die Translationsachse T drehbar und relativ zu dem Stützkörper 2 axial nicht bewegbar gelagert. Es weist in einem hinteren Bereich einen nach radial auswärts ragenden, umlaufenden Steg 22 auf, der mit einer Außenverzahnung versehen ist. Über die Außenverzahnung wird das Antriebsglied 20 um die Translationsachse T drehangetrieben. Seinen Drehantrieb erhält es von einem Drehmotor 18, der in einem weiteren Aufnahmeraum aufgenommen ist. Der weitere Aufnahmeraum wird von der Hüllstruktur 1 gebildet und ist von den beiden Aufnahmeräumen des Stützkörpers 2 getrennt. Der Motor 18 treibt das Abtriebsglied 20 über ein Zahnradgetriebe mit Stirnzahnrädern 19 an, von denen ein Ausgangszahnrad 19 mit der Außenverzahnung des Abtriebsglieds 20 kämmt. Infolge des Gewindeeingriffs zwischen dem Antriebsglied 20 und dem Abtriebsglied 10 und der Linearführung des Abtriebsglieds 10, wird bei einem Drehantrieb des Antriebsglieds 20 durch dessen rotatorische Antriebsbewegung eine axiale Abtriebsbewegung des Abtriebsglieds 10 in die Förderrichtung V bewirkt. Das durch die Kolbenbewegung verdrängte Produkt wird über den Katheter 8 ausgeschüttet und auf diese Weise verabreicht.

Wie jeder Gewindeeingriff ist auch der Gewindeeingriff als solcher zwischen dem Abtriebsglied 10 und dem Antriebsglied 20 mit einem Axialspiel behaftet. Die Dosiergenauigkeit der Ausschüttung ist daher zumindest im Ausmaß dieses inhärenten Axialspiels mit einer Ungenauigkeit behaftet. Im Falle beispielsweise eines Ansaugens des Kolbens 13 aufgrund Syphoning oder bei mechanischen Stößen oder Druckdifferenzen zwischen dem Gehäuseinnenraum und der Umgebung kann es daher vorkommen, dass die Flanken des Außengewindes 11 des Abtriebsglieds 10 von den vortreibenden Gewindeflanken des Gewindes 21 abheben. Die axiale Position des Kolbens 13 ist daher im Ausmaß des Axialspiels der Gewinde 11 und 21 unbestimmt.

Allerdings ist eine Spielreduziereinrichtung vorgesehen, die mittels eines Einstellglieds 25 gebildet ist und die das Axialspiel zwischen dem Abtriebsglied 10 und dem Antriebsglied 20 nahezu eliminiert.

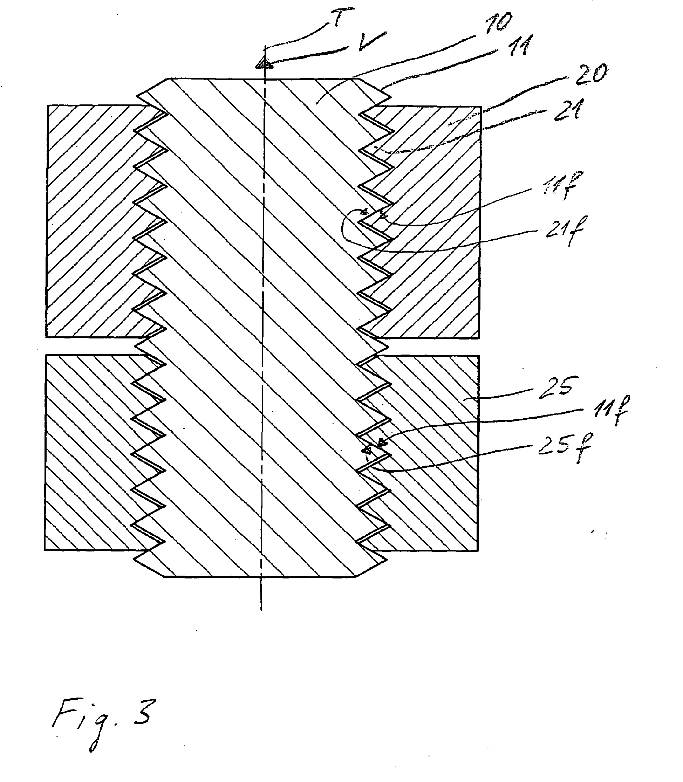

Aus den Figuren 2 und 3 sind Konstruktion und Wirkung der Spielreduziereinrichtung am besten ersichtlich. Die Spielreduziereinrichtung wird von einem einstückigen Einstellglied 25 gebildet. Das Einstellglied 25 ist in einem Einstelleingriff mit dem Abtriebsglied 10 und in einem weiteren Einstelleingriff mit dem Antriebsglied 20. Das Einstellglied 25 und die beiden Einstelleingriffe sind so gestaltet, dass das Axialspiel zwischen den Gewinden 11 und 21 deutlich reduziert oder vorzugsweise gänzlich aufgehoben wird.

Im Ausführungsbeispiel ist das Einstellglied 25 als Gewindemutter mit einem Innengewinde und einem Außengewinde gebildet. Mit seinem Innengewinde ist das Einstellglied 25 in einem Gewindeeingriff mit dem Außengewinde 11 des Abtriebsglieds 10. Mit seinem Außengewinde ist es in einem Gewindeeingriff mit einem Innengewinde 26 des Antriebsglieds 20. Das Innengewinde 26 ist dem Außengewinde 11 zugewandt und axial unmittelbar hinter dem Gewinde 21 gebildet. Das Innengewinde und das Außengewinde des Einstellglieds 25 liegen auf der gleichen axialen Höhe, so dass das Einstellglied 25 axial dünn sein kann und die Spielreduziereinrichtung dementsprechend axial, d. h. entlang der Translationsachse T, kurz baut. Das Innengewinde 26 muss lediglich so lang sein, dass ein sicherer Einstelleingriff mit dem Einstellglied 25 gewährleistet ist und das Einstellglied 25 darüber hinaus in diesem Einstelleingriff noch so weit verstellt werden kann, dass die angestrebte Reduzierung des Axialspiels der Gewinde 11 und 21 herbeigeführt werden kann. Das Innengewinde 26 weist eine Steigung auf, die es erlaubt, das Einstellglied 25 bei bestehendem Gewindeeingriff zwischen den Gewinden 11 und 21 und zwischen dem Gewinde 11 und dem Innengewinde des Einstellglieds 25 im Gewindeeingriff mit dem Innengewinde 26 zu verstellen.

Figur 3 zeigt in vergrößerter Darstellung den Gewindeeingriff der Gewinde 11 und 21 und den Einstelleingriff zwischen dem Gewinde 11 des Abtriebsglieds 10 und dem Innengewinde des Einstellglieds 25. Das Innengewinde des Einstellglieds 25 weist die gleiche Steigung wie das Außengewinde 11 auf. Vorzugsweise ist die Steigung des Außengewindes des Einstellglieds 25 und des Innengewindes 26 größer oder kleiner als die Steigung der Gewinde 11 und 21, allerdings so geringfügig, dass die Verstellung des Einstellglieds 25 im Einstelleingriff möglich ist.

Für die Axialspielreduzierung ist das Einstellglied 25 in seinem Einstelleingriff mit dem Abtriebsglied 10 so eingestellt, dass seine bezüglich der Förderrichtung V rückwärtigen Gewindeflanken 25f in Kontakt mit den vorderen Gewindeflanken 11 f des Außengewindes 11 sind, während gleichzeitig die vorderen Flanken 21 f des antreibenden Gewindes 21 in Kontakt mit den rückwärtigen Flanken 11 f des Gewindes 11 des Abtriebsglieds 10 sind. Hierfür wird das Einstellglied 25 in seinem Einstelleingriff mit dem Antriebsglied 20 relativ zu dem Antriebsglied 20 gegen die Förderrichtung V verstellt, bis dieser Zustand des Flankenkontakts hergestellt ist. In diesem Zustand wird das Einstellglied 25 an dem Antriebsglied 20 fixiert und dadurch gesichert. Die Sicherung wird im Ausführungsbeispiel adhäsiv hergestellt, indem ein Adhäsionsmittel in den Einstelleingriff des Einstellglieds 25 mit dem Antriebsglied 20 eingebracht ist. Andere Möglichkeiten der stoffschlüssigen Verbindung zwischen dem Einstellglied 25 und dem Antriebsglied 20 sind jedoch ebenfalls denkbar, beispielsweise eine Laserverschweißung in der Einstellposition. Falls das Innengewinde 26, wie bevorzugt, eine andere Steigung als die Gewinde 11 und 21 hat, kann bereits hierdurch allein oder in Kombination mit einer stoffschlüssigen Verbindung die axiale Sicherung erzielt werden. Die Einstellposition sollte so gewählt werden, dass durch die Spielreduzierung keine oder jedenfalls keine praktisch relevanten Presskräfte auf das Abtriebsglied 10 ausgeübt werden. Die Einstellposition ist daher so gewählt, dass in dem Gewindeeingriff der Gewinde 11 und 21 ein sehr geringes Restspiel verbleibt, das jedoch deutlich kleiner als das diesem Eingriff allein, d. h. ohne Spielreduzierung, inhärente Gewindespiel ist.

Im Ausführungsbeispiel ist die Einstellposition des Einstellglieds 25 so gewählt, dass das Gewinde 21 das Antriebsgewinde des Abtriebsglieds 20 bleibt. Die Einstellposition könnte jedoch auch so gewählt sein, dass bei der Einstellung das Einstellglied 25 gegen die rückwärtigen Gewindeflanken des Gewindes 11 bewegt wird und in diesem Fall das Einstellglied 25 den Vortrieb des Abtriebsglieds 10 übernimmt. Bevorzugt wird jedoch die für das Ausführungsbeispiel gewählte und aus Figur 3 ersichtliche Einstellposition des Einstellglieds 25.

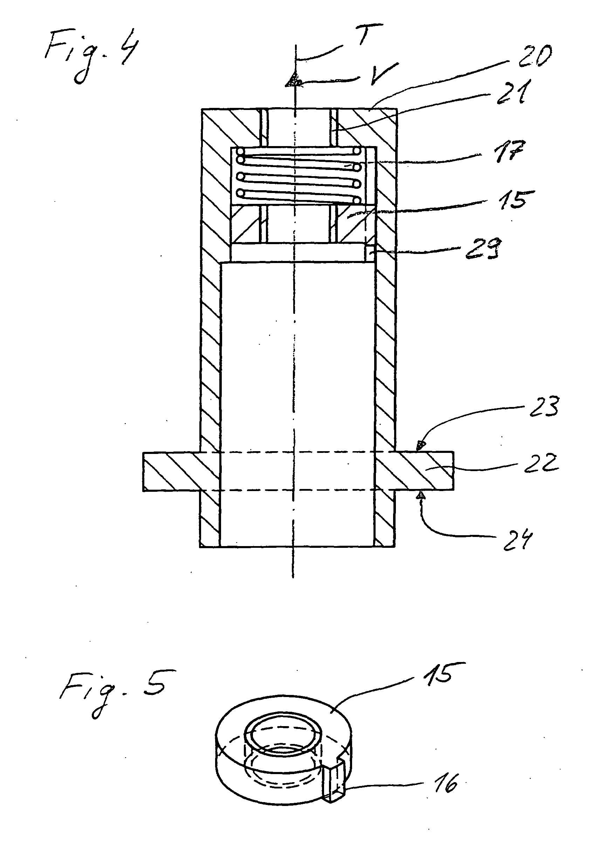

Ein alternatives Ausführungsbeispiel einer Spielreduziereinrichtung ist aus den Figuren 4 und 5 erkennbar. Im Vergleich zu den Komponenten des Ausführungsbeispiels der Figuren 1 bis 3 sind lediglich das Antriebsglied 20 und das im alternativen Ausführungsbeispiel mit "15" gekennzeichnete Einstellglied modifiziert, während die übrigen Komponenten, insbesondere das Abtriebsglied 10, unverändert sind. Als weiteren Unterschied umfasst die alternative Spielreduziereinrichtung zusätzlich ein elastisches Rückstellelement 17 in Form einer mechanischen Druckfeder.

Das Einstellglied 15 ist wie bereits das Einstellglied 25 in die das Abtriebsglied 20 bildende Hülse eingesetzt. Allerdings ist das Einstellglied 15 mit dem Abtriebsglied 20 axial linear verschiebbar und verdrehgesichert verbunden. Der Einstelleingriff des Einstellglieds 15 mit dem Antriebsglied 20 ist daher eine Linearführung. Die Linearführung wird von einer axialen, geraden Führungsbahn 29 an der Mantelinnenfläche des Antriebsglieds und einem in die Führungsbahn 29 eingreifenden Eingriffsglied 16 (Fig. 5) des Einstellglieds 15 gebildet. Die Führungsbahn 29 wird in die Förderrichtung V von dem nach radial einwärts ragenden Steg des Antriebsglieds 20 begrenzt, der das Antriebsgewinde 21 bildet. Rückwärtig ist die Führungsbahn 29 offen, so dass das Einstellglied 15 eingeschoben werden kann. Zuvor wird noch das Rückstellelement 17 eingesetzt. Das Rückstellelement 17 ist in Förderrichtung an dem das Antriebsgewinde 21 bildenden Steg des Antriebsglieds 20 und gegen die Förderrichtung an dem Einstellglied 15 abgestützt. Figur 4 zeigt diesen Zustand vor dem Zusammenbau mit dem Abtriebsglied 10.

Für den Zusammenbau wird zuerst das Einstellglied 15 mit dem Rückstellelement 17 in das Antriebsglied 20 in den Einstellgriff mit der Führungsbahn 29 eingesetzt und mit einer gewissen Kraft gegen das Rückstellelement 17 gedrückt. Das Abtriebsglied 10 wird anschließend zuerst mit dem Einstellglied 15 und dann mit dem Antriebsgewinde 21 verschraubt. Im Einstelleingriff drücken die rückwärtigen Gewindeflanken des Innengewindes des Einstellglieds 15 mit einer Elastizitätskraft gegen die in die Förderrichtung V weisenden Gewindeflanken des Gewindes 11 des Abtriebsglieds 10. Im Ergebnis wird für den Gewindeeingriff der Gewinde 11 und 21 durch die zwei Einstelleingriffe des Einstellglieds 15 der gleiche Zustand erhalten, wie er in Figur 3 dargestellt ist. In der alternativen Spielreduziereinrichtung wird das Einstellglied 15 somit durch die Elastizitätskraft des Rückstellelements 17 in der Einstellposition gesichert.

Das Infusionsgerät des Ausführungsbeispiels weist als eine Besonderheit, wie sie jedoch grundsätzlich aus der DE 198 40 992 A bekannt ist, eine Okklusionserkennung auf, der ebenfalls ein Axialspiel inhärent ist, unter dem die Dosiergenauigkeit leiden kann. Dieses inhärente, zweite Axialspiel hat seine Ursache darin, dass die gesamte Fördereinrichtung, insbesondere das Abtriebsglied 10 und das Antriebsglied 20 über einen Sensor 33 an dem Stützkörper 2 axial abgestützt ist. Der Sensor 33 dient der Ermittlung der Kraft, die erforderlich ist, um den Kolben 13 entlang der Translationsachse T zu bewegen. Der Sensor kann beispielsweise auf einer Dehnungsmessung beruhen. Letztlich wird mittels des Sensors 33 eine den Fluiddruck in dem Behältnis 12 repräsentierende, physikalische Größe gemessen, um bei der Produktverabreichung eine etwaige Okklusion oder ein etwaiges Leck möglichst frühzeitig zu erkennen. Was die Okklusions- und/oder Leckerkennung und den Sensor 33 anbetrifft, wird im Folgenden lediglich auf die das Axialspiel betreffenden Aspekte eingegangen und im Übrigen auf die als Beispiel in Bezug genommene DE 198 40 992 A verwiesen.

Für die Okklusions- und/oder Leckerkennung ist die Fördereinrichtung, wie bereits erwähnt, über den Sensor 33 axial abgestützt. Dies bedeutet, dass das Antriebsglied 20, an dem sich das Abtriebsglied 10 im Antriebseingriff der Gewinde 11 und 21 axial abstützt, nicht axial steif mit dem Stützkörper 2 verbunden, sondern relativ zu dem Stützkörper 2 axial bewegbar gelagert ist, um nämlich den Fluiddruck in dem Behältnis 12, genauer gesagt den Differenzdruck zur Umgebung, ermitteln zu können. Um die axial bewegbare Lagerung zu erhalten, ist das Antriebsglied 20 in einem Lagerkörper 30 drehgelagert und an dem Lagerkörper 30 axial gesichert. Der Lagerkörper 30 ist in den zweiten Aufnahmeraum des Stützkörpers 2 eingesetzt und gegen Verdrehung gesichert. Der Stützkörper 2 führt den Lagerkörper 30 axial durch Gleitkontakt. Der Lagerkörper 30, und damit zusammen das Abtriebsglied 10 und das Antriebsglied 20, ist über den Sensor 33 an dem Stützkörper 2 axial so abgestützt, dass der Sensor 33 die gesamte, zwischen dem Lagerkörper 30 und dem Stützkörper 2 wirkende und gegen die Förderrichtung V gerichtete Axialkraft aufnimmt. In Förderrichtung V ist der Lagerkörper 30 gegen den Stützkörper 2 auf Anschlag. Der Lagerkörper 30 lagert auch den Motor 18 und das Getriebe 19 der Fördereinrichtung. Im Ausführungsbeispiel ist er hierfür mit einem seitlichen Fortsatz gebildet, der durch eine seitliche Durchbrechung des Stützkörpers 2 in den seitlichen Aufnahmeraum der Gehäusehüllstruktur 1 ragt, in dem der Motor 18, eine Steuerung und gegebenenfalls ein weitergehendes Gerätemanagementsystem untergebracht sind. Der im Wesentlichen hohlzylindrische Stützkörper 2 ist für diesen Zweck in seinem Mantel mit einer Durchbrechung versehen, durch die auch der Sensor 33 mit einer Sensoranschlussseite ragt, an der er mit der Steuerung und einer Anzeige verbunden ist.

Der Sensor 33 bildet einen elastischen Biegebalken, der an beiden Enden fest eingespannt ist. Der Halterung des Sensors 33 dient ein als einstückig geformter Sensorträger 37, der von dem Stützkörper 2 gleitend axial geführt wird und über eine Kontaktstelle 6 und den Sensor 33 gegen den rückwärtigen Öffnungsrand des Lagerkörpers 30 auf Anschlag oder gegebenenfalls mit dem Lagerkörper 30 fest verbunden ist. Da der Sensorträger 37 auch dann jede Axialbewegung des Lagerkörpers 30 mitmacht, wenn lediglich Anschlagkontakt besteht, wird er dem Lagerkörper 30 und damit der Fördereinrichtung zugerechnet.

Um das Axialspiel zwischen dem Stützkörper 2 und dem Lagerkörper 30 zu eliminieren oder zumindest auf ein Maß zu reduzieren, das in Bezug auf die Dosiergenauigkeit tolerierbar oder nach Möglichkeit in der Praxis nicht mehr spürbar ist, ist ein Kontaktelement 5 vorgesehen, das als weiteres Einstellglied 5 dient. Das Einstellglied 5 bildet die Kontaktstelle 6 für den Sensor 33. Die Kontaktstelle 6 ist ein Nocken, der auf der Translationsachse T von der Vorderseite des Einstellglieds 5 in Förderrichtung V vorragt. Der Lagerkörper 30 ist über den Sensor 33 axial lediglich an der Kontaktstelle 6 quasi punktuell auf der Translationsachse T abgestützt.

Das Einstellglied 5 ist in einem Einstelleingriff mit dem Stützkörper 2. Auch dieser Einstelleingriff ist im Ausführungsbeispiel ein Gewindeeingriff, nämlich zwischen einem Innengewinde 4 an dem rückwärtigen Ende des Stützkörpers 2 und einem entsprechenden Außengewinde des Einstellglieds 5. Das Einstellglied 5 ist eine Kreiszylinderscheibe, die axial gerade so dick ist, dass sie für einen ausreichend festen Einstelleingriff an ihrem äußeren Umfang mit einem ausreichend langen Gewindezug versehen ist.

Die Einstellposition des Einstellglieds 5 ist so gewählt, dass der Lagerkörper 30 gegen den Stützkörper 2 in Förderrichtung auf Anschlag ist und gleichzeitig die Kontaktstelle 6 die Rückseite des Kraftsensors 33 gerade berührt. Das Innengewinde 4 des Stützkörpers 2 ist ausreichend lang, um den Einstellkörper 5 einzuschrauben und im Einstelleingriff bis in diese Einstellposition einstellen zu können. Die Einstellposition ist vorzugsweise so gewählt, dass eine Eichkurve des kalibrierten Sensors 33 nicht verändert wird, insbesondere dass der Nullpunkt der Eichkurve erhalten bleibt. Der Offset des Sensors 33 ist mit anderen Worten daher "Null"; wenn der Fluiddruck in dem Behältnis 12 dem Umgebungsdruck entspricht. Ein Offset wird erst bei einem Primen des Infusionsgeräts erhalten. Grundsätzlich kann die Einstellposition aber auch so gewählt sein, dass sich bereits in der Einstellposition ein Offset vor einem Primen einstellt. Dieser Einstelloffset sollte jedoch kleiner sein als der Offset, welcher sich bei einem Primen einstellt. Mit dem Begriff "Primen" wird der Vorgang bezeichnet, durch den die produktführenden Teile bis einschließlich zu einer Austrittsstelle des Katheters 8, der von einer Einstechkanüle oder einer weichen Kanüle gebildet werden kann, vollkommen mit dem Produkt gefüllt sind.

Das Einstellglied 5 ist in seiner Einstellposition an dem Stützkörper 2 festgelegt, vorzugsweise stoffschlüssig mit dem Stützkörper 2 verbunden. Der Stoffschluss kann beispielsweise durch Laserschweißung oder vorzugsweise durch ein in den Einstelleingriff eingebrachtes Adhäsionsmittel erhalten werden.

Auch ohne Okklusions- und/oder Leckerkennung ist ein Axialspiel nicht nur dem Gewindeeingriff zwischen dem Abtriebsglied 10 und dem Antriebsglied 20 inhärent, sondern auch den Drehlagerungen, wie sie von herkömmlichen Infusionsgeräten bekannt sind.

Um das Axialspiel in der Drehlagerung des Antriebsglieds 20 zu reduzieren, ist ein Einstellglied 35 vorgesehen. Das Einstellglied 35 ist in einem Einstelleingriff mit dem Lagerkörper 30. Auch dieser Einstelleingriff ist ein Gewindeeingriff. Wie bereits das Einstellglied 5 ist auch das Einstellglied 35 eine flache, scheibenförmige Schraube mit einem Außengewinde. Das Einstellglied 35 ist an dem rückwärtigen Ende des Lagerkörpers 30 in den Lagerkörper 30 eingeschraubt, der hierfür ein Innengewinde 34 bildet, das mit dem Außengewinde des Einstellkörpers 35 in dem Einstelleingriff ist. Das Einstellglied 35 ist so angeordnet, dass über die von der Drehlagerung ausgehenden Kräfte hinaus keine anderen äußeren Kräfte auf das Einstellglied 35 wirken können.

Für die axiale Abstützung und Festlegung des Antriebsglieds 20 bilden der Lagerkörper 30 eine gegen die Förderrichtung V weisende erste Stützfläche 31 und das Einstellglied 35 eine der Stützfläche 31 zugewandte zweite Stützfläche 32. Das Antriebsglied 20 bildet an seinem Steg 22 eine in die Förderrichtung V weisende dritte Stützfläche 23, die der ersten Stützfläche 31 zugewandt ist, und eine gegen die Förderrichtung V weisende vierte Stützfläche 24, die der zweiten Stützfläche 32 zugewandt ist. Zwischen den beiden Stützflächen 31 und 23 ist ein Kugellager 27 und zwischen den Stützflächen 32 und 24 ist ein weiteres Kugellager 28 axial gehalten. Jedes der Kugellager 27 und 28 bildet ein Radiallager und über die Stützflächen 31 und 23 sowie 32 und 24 ein Axiallager. Die Kugellager weisen wie üblich je einen inneren und einen äußeren Lagerring auf, die um die Translationsachse T relativ zueinander drehbar sind und zwischen denen je eine Mehrzahl von Kugeln angeordnet ist, die die Radial- und Axialkräfte zwischen den Lagerringen übertragen. Bei dem Kugellager 27 ist der innere Lagerring mit 27i und der äußere Lagerring mit 27a bezeichnet. Das Kugellager 28 weist entsprechend einen inneren Lagerring 28i und einen äußeren Lagerring 28a auf. Die inneren Lagerringe 27i und 28i sind radial an der äußeren Umfangsfläche des Abtriebsglieds 20 und die äußeren Lagerringe 27a und 28a sind radial an der gegenüberliegenden Mantelinnenfläche des Lagerkörpers 30 abgestützt.

Für die axiale Einspannung der Kugellager 27 und 28 ist das Einstellglied 35 in seiner Einstellposition mit einer geringen axialen Kraft gegen den äußeren Lagerring 28a gepresst. Das Einstellglied 35 und das Kugellager 28 sind nur mit dem äußeren Lagerring 28a und der zweiten Stützfläche 32 in Kontakt. Die zweite Stützfläche 32 ist eine geschlossen umlaufende, zu der Rotationsachse T konzentrische Ringstirnfläche eines Ringstegs 36, der in die Förderrichtung V von der Vorderseite des Einstellglieds 35 aufragt. Die Ringstirnfläche könnte auch Unterbrechungen aufweisen. Ebenso könnte die Stützfläche 32 durch einzeln aufragende Nocken gebildet sein.

Die äußeren Lagerringe 27a und 28a haben axial keinen Kontakt mit den Stützflächen 23 und 24 des Antriebsglieds 20. Die inneren Lagerringe 27i und 28i haben axial keinen Kontakt mit den Stützflächen 31 und 32. Der axiale Kraftfluss verläuft durch die Drehlagerung daher ausschließlich über den Kontakt der Stützflächen 31 und 32 mit dem jeweils zugewandten der äußeren Lagerringe 27a und 28a und den Kontakt zwischen den Stützflächen 23 und 24 und dem jeweils zugewandten der inneren Lagerringe 27i und 28i. Die axiale Kraft wird innerhalb der Kugellager 27 und 28 deshalb ausschließlich durch die Kugeln übertragen. Auf diese Weise entsteht abgesehen von Fertigungstoleranzen der Kugellager 27 und 28 eine axial nahezu spielfreie Drehlagerung.

Das Einstellglied 35 wird bereits wie die zuvor beschriebenen Einstellglieder in seiner Einstellposition gesichert. Die Sicherung an dem Lagerkörper 30 ist ebenfalls vorzugsweise stoffschlüssig und kann insbesondere durch ein Adhäsionsmittel bewirkt werden, das in den Einstelleingriff eingebracht ist. Andere Stoffschlussverbindungen, beispielsweise Laserschweißung, sind jedoch ebenfalls möglich. Wie bei den anderen Einstellgliedern wird die Sicherung im Einstelleingriff selbst vorgenommen.

Zu den Einstelleingriffen ist noch zu bemerken, dass die axialen Längen der Verstellwege der Einstellglieder 5, 15, 25 und 35 in den Einstellgriffen je so lang sind, dass das jeweilige Einstellglied bei der Verstellung in die Einstellposition nicht gegen den Körper, mit dem es in dem Einstelleingriff ist, in einen Anschlagkontakt gelangen kann, der eine weitere Verstellung gleicher Richtung blockiert.

Noch eine weitere Quelle für ein der Dosiergenauigkeit abträgliches Axialspiel ist bei herkömmlichen Infusionsgeräten und auch bei herkömmlichen Injektionsgeräten der große Unterschied zwischen der axialen Wärmeausdehnung der Gehäuse und der axialen Wärmeausdehnung der eingesetzten Reservoirbehältnisse. Die Gehäuse sind üblicherweise aus Kunststoff im Spritzguss hergestellt, während die Behältnisse meist Glaskörper sind. Die Wärmeausdehnungskoeffizienten dieser Materialien unterscheiden sich im Allgemeinen etwa um den Faktor 10, d. h. um eine ganze Größenordnung. Für den axialen Ausgleich dieser Unterschiede in der Wärmeausdehnung sind die Behältnisse bei den herkömmlichen Geräten axial elastisch nachgiebig an den Gehäusen abgestützt. Im Einsatztemperaturbereich der Geräte, der immerhin den Bereich von -20°C bis 40°C abdeckt, ändern sich daher die Positionen zwischen den Fördereinrichtungen und den Behältnissen axial in einem Ausmaß, das die Dosiergenauigkeit spürbar beeinträchtigt.

Diesem Axialspiel und seiner für die Dosiergenauigkeit negativen Wirkung wird dadurch entgegengewirkt, dass der Stützkörper 2 in Axialrichtung eine Wärmeausdehnung aufweist, die deutlich näher bei der axialen Wärmeausdehnung des Behältnisses 12 liegt als die Gehäuse von herkömmlichen Geräten. So kann der Stützkörper 2 insbesondere aus einem Material bestehen, dessen Wärmeausdehnungskoeffizient sich höchstens um einen Faktor 5 von dem Wärmeausdehnungskoeffizient des Materials des Behältnisses 12 unterscheidet. Bevorzugter ist es, wenn die Wärmeausdehnungskoeffizienten so nah als möglich beieinander liegen oder gar gleich sind. Auch konstruktive Maßnahmen sind denkbar, beispielsweise die Fertigung des Stützkörpers 2 als Verbundkörper, der im Verbund mehrere Materialien aufweist, beispielsweise in Kunststoff eingelegte Aussteifungskörper, die die Wärmeausdehnung des Kunststoffinaterials in axialer Richtung behindern. Bevorzugte Materialien für den Erhalt einer günstigen Wärmeausdehnung weisen im Einsatztemperaturbereich einen Wärmeausdehnungskoeffizienten von 30 x 10⁻⁶ / K oder weniger auf. Die Materialien weisen vorzugsweise eine in alle Richtungen gleichmäßige Wärmeausdehnung auf. Eine Stützstruktur in Form eines Verbundkörpers wird allerdings naturgemäß eine ungleichmäßige Wärmeausdehnung, bezogen auf den gesamten Verbundkörper, aufweisen, so dass in solch einem Fall nur die axiale Wärmeausdehnung und der axiale Wärmeausdehnungskoeffizient gemeint sind.

Bevorzugte Konstruktionsmaterialien für Infusionsgeräte und Injektionsgeräte sind zusammen mit deren im Einsatztemperaturbereich geltenden Wärmeausdehnungskoeffizienten α in der folgenden Tabelle zusammengestellt:

| Material | Wärmeausdehnungskoeffizient α in 10⁻⁶ / K |
|---|---|
| Messing | 18 bis 19 |
| Stahl | 10 bis 12 |
| Aluminium | 23 bis 24 |
| Polyamid PA | 100 bis 140 |
| Polyoxymethylen POM | 110 bis 130 |
| Polyethylenterephthalat PET | 70 |
| Polycarbonat PC | 70 |
| Polytetrafluorethylen PTFE | 60 bis 200 |
| Acrylnitril/Butadien/Styrol ABS | 80 bis 110 |
| Glas | 5 bis 10 |
| Hartgummi | 75 bis 100 |

Durch einen Stützkörper 2 oder allgemeiner eine Stützstruktur 2 aus beispielsweise Aluminium oder einer Aluminium-Basislegierung kann bereits gegenüber denjenigen Kunststoffmaterialien, die in Bezug auf die Wärmeausdehnung dem Behältnismaterial, vorzugsweise Glas, am nächsten kommen, eine deutliche Verbesserung erzielt werden, da der Wärmeausdehnungskoeffizient von Aluminium um etwa den Faktor 3 kleiner ist als der Wärmeausdehnungskoeffizient der den Behältnismaterial in Bezug auf den Wärmeausdehnungskoeffizient am nächsten kommenden Kunststoffinaterialien. Durch die Verwendung eines Messingmaterials ist eine weitere Verbesserung erzielbar. Wird die Stützstruktur aus Stahl gebildet oder weist sie Stahlkomponenten in solch einer Anordnung auf, dass durch die Stahlkomponenten die axiale Wärmeausdehnung maßgeblich beeinflusst wird, so kann bei entsprechender Wahl des Glasmaterials im günstigsten Fall sogar eine identische Wärmeausdehnung erzielt werden. Wird der Stützkörper 2 oder allgemeiner eine Stützstruktur 2, die natürlich ebenfalls axiale Stützfunktion wie der Stützkörper 2 übernimmt, als Verbundkörper gebildet, so kann durch Aussteifungskörper, beispielsweise in eine Kunststoffmatrix eingelagerte Axialfasern, ein vergleichbar günstiges Wärmeausdehnungsverhalten erzielt werden, wenn der oder die Aussteifungskörper eine Wärmeausdehnung wie vorstehend beschrieben aufweisen.

Auf die Mehrteiligkeit des Gehäuses, im Ausführungsbeispiel Zweiteiligkeit, kann grundsätzlich sogar verzichtet werden, wenn die Gehäusehülle, im Ausführungsbeispiel die Hüllstruktur 1, eine erfindungsgemäße Wärmeausdehnung besitzt. Bei solch einer Ausbildung einer Gehäusehülle wird es bevorzugt, wenn die Gehäusehülle als Verbundkörper gebildet ist, beispielsweise als Kunststoffmatrix mit eingelegten Aussteifungskörpern wie insbesondere axial orientierte Metallfasern.

Wenngleich bereits allein eine Stützstruktur vorteilhaft ist, die nur das Behältnis axial abstützt, ist es vorteilhafter, wenn solch eine Stützstruktur sich über eine möglichst große, in Förderrichtung V des Kolbens 13 gemessene Länge erstreckt. Die Stützstruktur sollte, wie beispielhaft der Stützkörper 2, zusätzlich auch noch die Fördereinrichtung axial in beide Richtungen abstützen. Besonders günstig ist es ferner, wenn auch die Fördereinrichtung im Ganzen eine axiale Wärmeausdehnung aufweist, die möglichst nah bei der axialen Wärmeausdehnung der Stützstruktur angesiedelt ist, beispielsweise indem die Stützstruktur und die Komponenten der Fördereinrichtung aus dem gleichen Material oder aus gegebenenfalls unterschiedlichen Materialien mit möglichst nahe beieinander liegender axialer Wärmeausdehnung gefertigt sind. Vorteilhafterweise weist das Abtriebsglied 10 oder das Antriebsglied 20 oder weisen vorzugsweise diese beiden Komponenten je eine im wesentlichen gleiche axiale Wärmeausdehnung wie der Stützkörper 2 auf, d.h. eine Wärmeausdehnung, die sich um höchstens den Faktor 5 und bevorzugter um weniger als den Faktor 5, möglichst um höchstens den Faktor 2 oder noch weniger von der axialen Wärmeausdehnung des Stützkörpers 2 unterscheidet und idealerweise gleich ist.

Je größer die von der bevorzugt einteiligen Stützstruktur oder den mehreren Stützkörpern einer mehrteiligen Stützstruktur erst gemeinsam überbrückte axiale Länge ist, desto geringer ist das auf unterschiedliche axiale Wärmeausdehnungen zurückzuführende Axialspiel. Kunststoffteile herkömmlicher Art dürfen hierbei durchaus kurze axiale Längen überbrücken. Je kürzer die von herkömmlichen Kunststoffteilen überbrückten axialen Längen sind, desto geringer ist das auf unterschiedliche Wärmeausdehnungen zurückzuführende Axialspiel. Besonders günstig ist es, wie im Ausführungsbeispiel, wenn solch ein Stützkörper oder gegebenenfalls auch mehrere axial hintereinander angeordnete Stützkörper vorgesehen ist oder sind, der oder die bezüglich ihrer axialen Wärmeausdehnung nahe bei derjenigen des Behältnisses und/oder der Fördereinrichtung liegen. Die Stützeinrichtungen des Stützkörpers, die das Behältnis und/oder die Fördereinrichtung axial an dem Stützkörper oder den Stützkörpern festlegen, sollten von jeweiligen dem Stützkörper in einem Stück gebildet oder mit dem jeweiligen Stützkörper so verbunden sein, dass sie relativ zu dem Stützkörper axial nicht bewegbar sind, wie beispielsweise das Paar der Stege 3 und 7a, das Paar aus Stützsteg 3 und Einstellglied 5 und das Paar aus Einstellglied 35 und Stützfläche 31.

Der Stützkörper 2 ist ein vergleichsweise einfacher Hülsenkörper, der in die Hüllstruktur 1 eingesetzt ist und lediglich der Lagerung der relativ zueinander axial bewegbaren Teile und damit zur axialen Aussteifung vorgesehen ist. Die Hüllstruktur 1 selbst kann wie üblich aus Kunststoff im Spritzguss hergestellt sein. Die Hüllstruktur 1 besteht aus zwei Teilen, nämlich einem Hauptteil und einem Bodenteil. Das Hauptteil bildet die Aufnahmekammer für die Stützstruktur 2 und die gegebenenfalls nicht von der Stützstruktur 2 gelagerten Komponenten der Verabreichungsvorrichtung. Das Bodenteil ist eine einfache Platte, die mit dem Hauptteil an dessen Rückseite fest verbunden ist und dort die Aufnahmekammer verschließt.

Der Deckel 7 besteht vorzugsweise aus dem gleichen Material wie der Stützkörper 2. Ebenso gilt dies für den Lagerkörper 30, die beiden Einstellglieder 35 und 5 und die Trägerscheibe 37, so dass eine insgesamt in Bezug auf die axiale Wärmesausdehnung sehr homogene Stützstruktur erhalten wird. Eine Fertigung des Deckels 7 und/oder der Trägerscheibe 37 und/oder des Einstellglieds 35 und/oder des Einstellglieds 5 aus einem der üblichen Kunststoffmaterialien ist jedoch weit weniger kritisch als die Bildung einer axial langen Stützstruktur aus den herkömmlichen Kunststoffmaterialien.

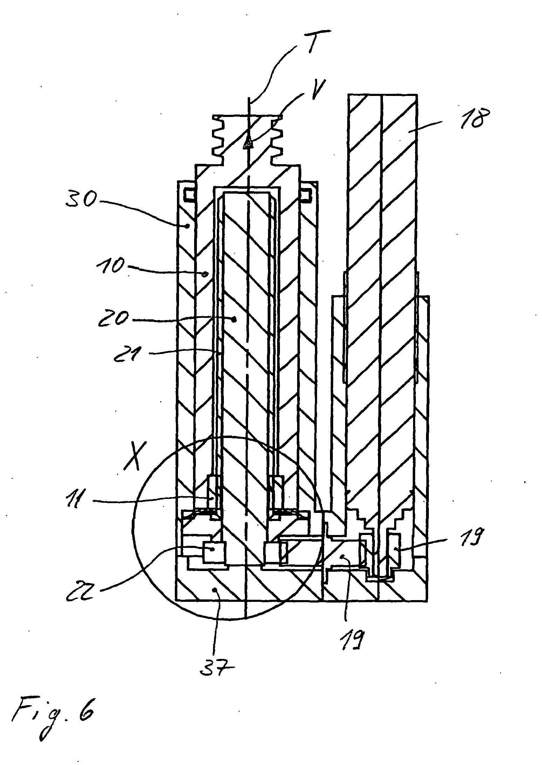

Figur 6 zeigt in einem Längsschnitt einen wie im Ausführungsbeispiel gelagerten Lagerkörper 30 mit den von ihm gelagerten Komponenten einer zweiten Verabreichungsvorrichtung die ebenfalls ein Infusionsgerät ist. Diejenigen Komponenten der zweiten Verabreichungsvorrichtung, die ihrer Funktion und zum Teil auch Konstruktion nach mit Komponenten des Ausführungsbeispiels vergleichbar sind, tragen die gleichen Bezugszeichen wie im Ausführungsbeispiel. Unterschiede bestehen nur soweit nachfolgend hierauf aufmerksam gemacht wird oder aus den Figuren selbst sich ergibt. Es sollen die Ausführungen zum Ausführungsbeispiel gelten, soweit sich hieraus keine offensichtlichen Widersprüche ergeben.

Die zweite Verabreichungsvorrichtung, die wicht teil den Erfindung ist, weist eine Spielreduziereinrichtung lediglich für die Eliminierung oder zumindest Reduzierung des Axialspiels zwischen dem Rotationsglied 20 und dem Lagerkörper 30 auf. Die Spielreduziereinrichtung spannt das Rotationsglied 20 im Unterschied zum Ausführungsbeispiel axial unmittelbar gegen den Sensorträger 37. Ferner umgibt das Translationsglied 10 das Rotationsglied 20. Das Translationsglied 10 und das Rotationsglied 20 sind miteinander in einem Gewindeeingriff. Hierfür ist das Rotationsglied 20 über den größten Teil seiner axialen Länge mit einem Außengewinde 21 und das Translationsglied 10 nur an seinem bezüglich der Translationsrichtung V rückwärtigen Ende mit einem Innengewinde 11 versehen. Das Translationsglied 10 ist an dem Lagerkörper 30 axial linear geführt. Wie beim Ausführungsbeispiel treibt ein Motor 18, vorzugsweise ein elektrischer Schrittmotor, über ein Stirnradgetriebe mit zwei in Stirneingriff befindlichen Zahnrädern 19 das Rotationsglied 20 um die Rotations- und Translationsachse T rotatorisch an. Das Rotationsglied 20 ist für seinen Antrieb an seinem rückwärtigen Ende wieder mit einem umlaufenden außenverzahnten Ringsteg 22 versehen, der mit dem Zwischenrad 19 des Stirnradgetriebes für den rotatorischen Antrieb des Rotationsglieds 20 in einem Stirneingriff ist.

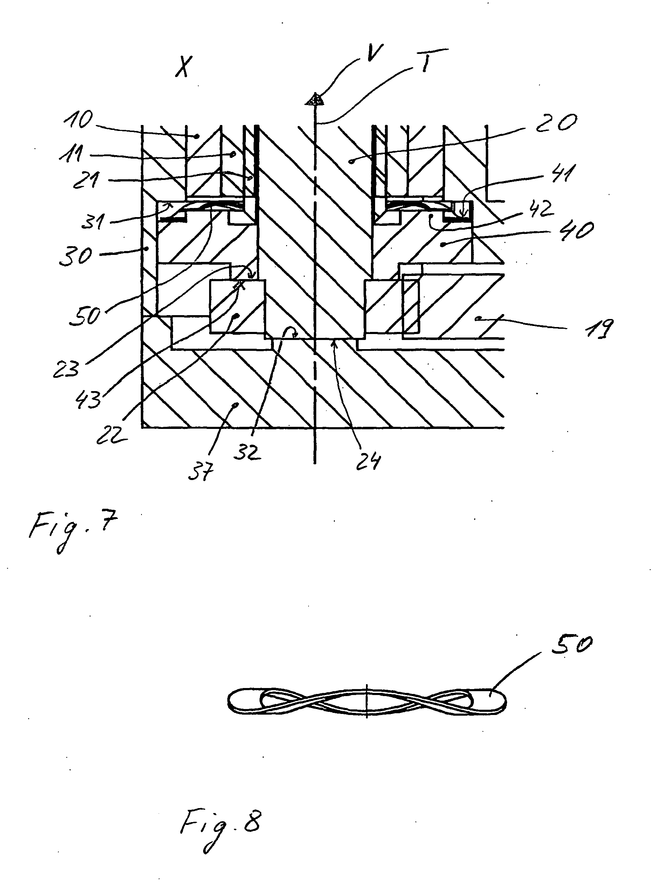

Die Drehlagerung des Rotationsglieds 20 ist in Figur 7 vergrößert dargestellt. Die Drehlagerung ist als einfache Gleitlagerung gebildet. Die zweite Stützfläche 32 des Sensorträgers 37 und die vierte Stützfläche 24 des Rotationsglieds 20 bilden ein erstes Gleitpaar der Drehlagerung. Die beiden Stützflächen 32 und 24 befinden sich unmittelbar miteinander in Gleitkontakt. Die zweite Stützfläche 32 ist an dem rückwärtigen Stirnende des Rotationsglieds 20 gebildet. Von dem Sensorträger 37 ragt ihr entgegen ein kurzer Sockel auf, dessen Stirnfläche die zweite Stützfläche 24 bildet. Der Sockel stellt das Rotationsglied 20 von dem Sensorträger 37 frei. Die Formung eines Sockels ermöglicht eine präzisere Fertigung der zweiten Stützfläche 32. Die dritte Stützfläche 23 ist in der Art wie die Stützfläche 23 des Ausführungsbeispiels gebildet, nämlich durch die in Translationsrichtung T weisende vordere Stirnfläche des Ringstegs 22. Die ihr axial zugewandte erste Stützfläche 31 wird von dem Lagerkörper 30 gebildet. Allerdings sind die Stützflächen 31 und 23 radial zueinander versetzt, d.h. sie liegen axial nicht exakt in der Flucht. Der Radialversatz wird von einem Übertragungskörper 40,einer ein Übertragungsring, überbrückt, der zwischen den Stützflächen 31 und 23 angeordnet ist. Der Übertragungskörper 40 bildet an einer vorderen Stirnseite der ersten Stützfläche 31 axial in der Flucht gegenüberliegend eine um die Translationsachse T und das Rotationsglied 20 umlaufende vordere Stützfläche 41 und an seiner Rückseite der dritten Stützfläche 23 axial in der Flucht gegenüberliegend eine rückwärtige Stützfläche 43. Die rückwärtige Stützfläche 43 ist unmittelbar auf Anschlagkontakt mit der dritten Stützfläche 23. Zwischen der ersten Stützfläche 31 und der ihr zugewandten vorderen Stützfläche 41 verbleibt ein lichter, axialer Abstand. Zwischen den beiden Stützflächen 31 und 41 ist eine Ringfeder 50 angeordnet, die sich axial an beiden Stützflächen 31 und 41 mit einer axialen Vorspannkraft abstützt.

Die Ringfeder 50 ist in Figur 8 einzeln dargestellt. Sie ist umlaufend wellenförmig und beispielsweise aus Federstahl gefertigt. Im eingebauten Zustand stützt sie sich mit ihren Wellenbergen und Wellentälern umlaufend alternierend an der ersten Stützfläche 31 und der vorderen Stützfläche 41 des Übertragungskörpers 40 ab. Bei axialer Stauchung wirkt sie wie eine Blattfeder.

Wie insbesondere in Figur 7 erkennbar, dient der Übertragungskörper 40 nicht nur dem Ausgleich des Radialversatzes, sondern auch der Zentrierung der Ringfeder 40. Hierfür ist der Übertragungskörper 40 an seiner vorderen Stirnseite mit einem umlaufenden Vorsprung 42 versehen, um dessen äußeren Umfang die vordere Stützfläche 41 axial ein Stück weit zurückgenommen umläuft.

Die Ringfeder 50 und der Übertragungskörper 40 bilden die Spielreduziereinrichtung , indem der Übertragungskörper 40 relativ zu dem Lagerkörper 30 axial bewegbar ist. Vorzugsweise wird er von dem Lagerkörper 30 axial linear geführt. Grundsätzlich kann er jedoch relativ zu dem Lagerkörper 30 drehbeweglich sein. Obgleich der Übertragungskörper 40 grundsätzlich mit dem Rotationsglied 20 drehsteif verbunden sein kann, entweder mittels Fügeverbindung oder durch eine einstückige Ausbildung mit dem Rotationsglied 20, wird es bevorzugt, wenn der Übertragungskörper 40 relativ zu dem Rotationsglied 20 drehbeweglich und noch bevorzugter auch axial beweglich angeordnet ist. Auf diese Weise wird ein weiteres Gleitflächenpaar der Drehlagerung durch die unmittelbar aneinander abgleitenden Stützflächen 23 und 43 gebildet. Die Ringfeder 50 wird somit vorteilhafterweise von Drehbewegungen freigehalten.

Bei der zweiten Verabreichungsvorrichtung wird im Ergebnis eine einfache Spielreduziereinrichtung für das Rotationsglied 20 erhalten, die bei ausreichender Vorspannung der Ringfeder 50 jegliches Axialspiel zwischen dem Rotationsglied 20 und dem Lagerkörper 30 eliminiert. In Ausführungen, in denen die Stützflächen 31 und 23 axial einander in der Flucht gegenüberliegen, könnte auf den Übertragungskörper 40 verzichtet werden. Um die Ringfeder 50 jedoch auch in solchen Ausführungen von Drehbewegungen freizuhalten und/oder eine einfach fertigbare Zentrierung für die Ringfeder 50 oder auch eine die Presskraft erzeugende andere Federeinrichtung zu erhalten, ist die Zwischenschaltung eines Übertragungskörpers in der Art des Übertragungskörpers 40 auch dann von Vorteil.

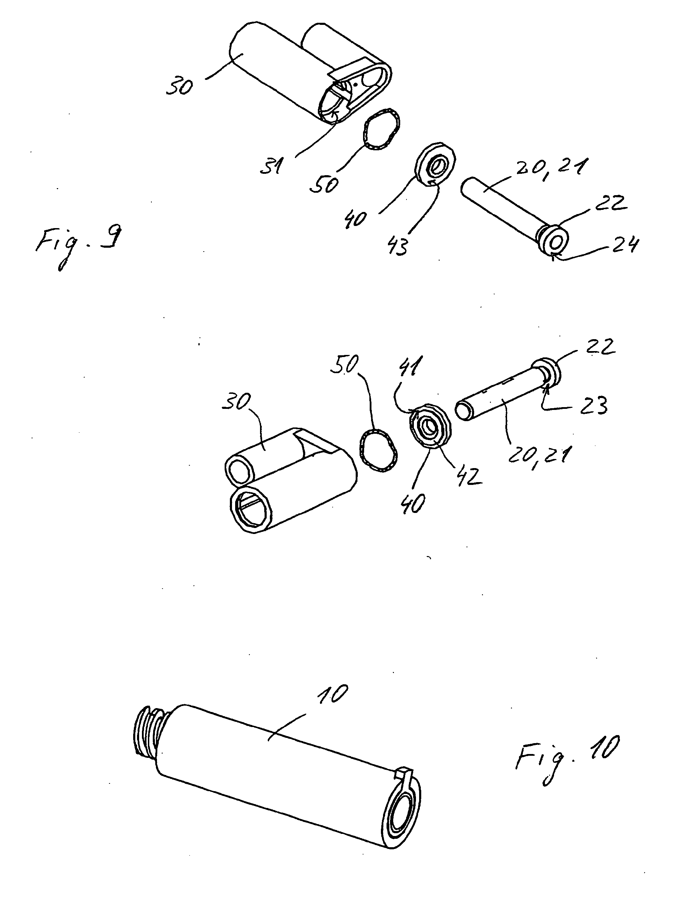

Figur 9 zeigt in einer Explosionsdarstellung den Lagerkörper 30, das Rotationsglied 20, den Übertragungskörper 40 und die Ringfeder 50 entlang der zu denkenden Translationsachse in einer für die Montage geeigneten Folge aufgereiht. Figur 10 zeigt das Translationsglied 10 einzeln. Bei der Montage kann in einem ersten Schritt das Translationsglied 10 alleine von hinten in den Lagerkörper eingesetzt und anschließend das Rotationsglied 20 in das Translationsglied 10 eingeschraubt werden, oder es kann zuerst die Gewindeverbindung zwischen dem Translationsglied 10 und dem Rotationsglied 20 hergestellt und erst anschließend das Translationsglied 10 mit dem eingeschraubten Rotationsglied 20 in den Lagerkörper 30 eingesetzt werden. Vor dem Einschrauben des Rotationsglieds 20 werden der Übertragungskörper 40 und die Ringfeder 50 über das Außengewinde 21 bis gegen den Ringsteg 22 des Rotationsglieds 20 geschoben, und anschließend wird das Rotationsglied 20 in das Translationsglied 10 eingeschraubt. Nachdem das Translationsglied 10 und das Rotationsglied 20 in dem Lagerkörper 30 angeordnet sind, wird der Sensorträger 37 mit dem in Figur 9 gezeigten Hauptteil des Lagerkörpers 30 verbunden, so dass es die zum Zwecke der Montage offene Rückseite des Lagerkörpers 30 verschließt. Lagerkörper 30 und Sensorträger 37 sind im verbundenen Zustand relativ zueinander nicht bewegbar. Die Verbindung ist ferner so gestaltet, dass die Ringfeder 50 mit einer definierten axialen Vorspannkraft eingebaut ist.

Der Gewindeeingriff der Gewinde 11 und 21 ist als einfacher Gewindeeingriff gebildet, kann jedoch ohne weiteres wie beim Ausführungsbeispiel mittels einer zusätzlich Spielreduziereinrichtung axialspielreduzierend gebildet sein.

Schließlich sei auch darauf hingewiesen, dass die auf dem verstellbaren Einstelleingriff beruhende Spielreduziereinrichtung 35 bei der zweiten Verabreichungsvorrichtung statt der Spielreduziereinrichtung 40, 50 vorgesehen sein kann. Auch Mischformen sind denkbar. So könnte eines der Wälzlager 27 und 28 zwischen einem der Stützflächenpaare 31, 23 und 32, 24 oder je solch ein Wälzlager zwischen beiden Stützflächenpaaren angeordnet sein, wobei das oder die beiden Wälzlager vorzugsweise je wie die Wälzlager 27 und 28 des ersten Ausführungsbeispiels angeordnet wären, d.h. es würde die Ringfeder 50 oder eine alternative Feder nur auf eine der Lagerschalen solch eines Wälzlagers wirken.

Der Lagerkörper 30 kann unter Verzicht auf die axial bewegliche Lagerung und den Sensor 33 zu einem Gehäuse mit einer Aufnahme für ein Reservoir 12 abgewandelt werden und dann direkt als Hüllstruktur wie die Hüllstruktur des Ausführungsbeispiels dienen. Solch eine Hüllstruktur kann wie herkömmliche Gehäuse gebildet sein. Alternativ kann sie jedoch die Wärmeausdehnungseigenschaften der Stützstruktur 2 des Ausführungsbeispiels haben, so dass auf die diesbezüglichen Ausführungen zum Ausführungsbeispiel verwiesen wird.

### Bezugszeichen:

- 1: Gehäusestruktur, Hüllstruktur
- 2: Gehäusestruktur, Stützstruktur, Stützkörper
- 3: Stützschulter, Stützsteg
- 4: Gewinde
- 5: Einstellglied, Kontaktelement
- 6: Kontaktstelle
- 7: Abschlusselement, Deckel
- 7a: Gegenstützschulter, Gegenstützsteg
- 8: Katheter
- 9: Eingriffseinrichtung, Gewinde
- 10: Abtriebsglied, Translationsglied
- 11: Gewinde
- 11f: Eingriffsflanke
- 12: Reservoir, Behältnis
- 13: Kolben
- 14: Auslass
- 15: Einstellglied
- 16: Eingriffsglied
- 17: Rückstellelement
- 18: Motor
- 19: Zahnräder
- 20: Antriebsglied, Rotationsglied
- 21: Gewinde
- 21f: Eingriffsflanke
- 22: Steg
- 23: Stützfläche
- 24: Stützfläche
- 25: Einstellglied
- 25f: Eingriffsflanke
- 26: Gewinde
- 27: Wälzlager
- 27a: äußerer Lagerring
- 27i: innerer Lagerring
- 28: Wälzlager
- 28a: äußerer Lagerring
- 28i: innerer Lagerring
- 29: Führungsbahn
- 30: Lagerkörper
- 31: Stützfläche
- 32: Stützfläche
- 33: Sensor
- 34: Gewinde
- 35: Einstellglied
- 36: Steg
- 37: Sensorträger
- 38: -
- 39: -
- 40: Übertragungskörper
- 41: vordere Stützfläche
- 42: Vorsprung
- 43: rückwärtige Stützfläche
- 44-49: -
- 50: Feder

- T: Translationsachse, Rotationsachse
- V: Förderrichtung

## Patentansprüche

1. Vorrichtung für die dosierte Verabreichung eines fluiden Produkts, die Vorrichtung umfassend:
a) ein Gehäuse (1, 2),
b) ein von dem Gehäuse (1, 2) aufgenommenes oder gebildetes Reservoir (12) für das Produkt,
c) ein Antriebsglied (20), das eine Antriebsbewegung ausführt und wenigstens eine Eingriffsflanke (21f) aufweist,
d) ein Abtriebsglied (10), das eine axiale Abtriebsbewegung ausführt, um Produkt aus dem Reservoir (12) zu fördern, und das wenigstens eine Eingriffsflanke (11f) aufweist, die mit der Eingriffsflanke (21f) des Antriebsglieds (20) in solch einem Flankeneingriff ist, dass die Antriebsbewegung des Antriebsglieds (20) die Abtriebsbewegung des Abtriebsglieds (10) bewirkt,
e) und eine Spielreduziereinrichtung (25; 15, 17), die in einem Einstelleingriff mit dem Antriebsglied (20) und dem Abtriebsglied (10) relativ zu dem Antriebsglied (20) und dem Abtriebsglied (10) in solch eine Einstellposition verstellt und in der Einstellposition so gesichert ist, dass ein Axialspiel des Flankeneingriffs reduziert ist,
f) **dadurch gekennzeichnet, dass** die Spielreduziereinrichtung (25; 15, 17) eine von dem Antriebsglied (20) und dem Abtriebsglied (10) separate Einrichtung ist, die wenigstens ein Einstellglied (25; 15) aufweist.

2. Vorrichtung nach Anspruch 1, **dadurch gekennzeichnet, dass** das Abtriebsglied (10) weitere Eingriffsflanken (11f) aufweist, die für die Förderung nacheinander mit der wenigstens einen Eingriffsflanke (21f) des Antriebsglieds (20) in Flankeneingriff gelangen und dass die Spielreduziereinrichtung (25; 15, 17) wenigstens eine Eingriffsflanke (25f) aufweist, die mit einer der weiteren Eingriffsflanken (11f) des Abtriebsglieds (10) in einem den Einstelleingriff mit dem Abtriebsglied (10) bildenden Flankeneingriff sind.

3. Vorrichtung nach einem der vorhergehenden Ansprüche, **dadurch gekennzeichnet, dass** der Flankeneingriff des Antriebsglieds (20) mit dem Abtriebsglied (10) ein Gewindeeingriff ist.

4. Vorrichtung nach einem der vorhergehenden Ansprüche, **dadurch gekennzeichnet, dass** der Einstelleingriff der Spielreduziereinrichtung (25; 15, 17) mit dem Abtriebsglied (10) ein Gewindeeingriff ist.

5. Vorrichtung nach einer Kombination der zwei vorhergehenden Ansprüche, **dadurch gekennzeichnet, dass** das Abtriebsglied (10) ein Gewinde (11) aufweist, mit dem es den Gewindeeingriff mit dem Antriebsglied (20) und den Gewindeeingriff mit der Spielreduziereinrichtung (25; 15, 17) bildet.

6. Vorrichtung nach einem der vorhergehenden Ansprüche, **dadurch gekennzeichnet, dass** das Abtriebsglied (10) eine Gewindestange ist.

7. Vorrichtung nach einem der vorhergehenden Ansprüche, **dadurch gekennzeichnet, dass** die Spielreduziereinrichtung (25; 15, 17) ein in sich axial steifes Einstellglied (25; 15) ist oder aufweist, das in dem Einstelleingriff mit dem Antriebsglied (20) und in dem Einstelleingriff mit dem Abtriebsglied (10) ist.

8. Vorrichtung nach einem der vorhergehenden Ansprüche, **dadurch gekennzeichnet, dass** der Einstelleingriff zwischen dem Antriebsglied (20) und der Spielreduziereinrichtung (25) ein Gewindeeingriff ist.

9. Vorrichtung nach einer Kombination der zwei vorhergehenden Ansprüche, **dadurch gekennzeichnet, dass** die Spielreduziereinrichtung (25) ein Innengewinde und ein Außengewinde aufweist und mit dem einen der Gewinde in dem Einstelleingriff mit dem Abtriebsglied (10) und mit dem anderen der Gewinde in dem Einstelleingriff mit dem Antriebsglied (20) ist.

10. Vorrichtung nach einem der vorhergehenden Ansprüche, **dadurch gekennzeichnet, dass** die Spielreduziereinrichtung (25) in der Einstellposition stoffschlüssig gesichert ist.

11. Vorrichtung nach dem vorhergehenden Anspruch, **dadurch gekennzeichnet, dass** die Spielreduziereinrichtung (25) stoffschlüssig an dem Antriebsglied (20) gesichert ist.

12. Vorrichtung nach einem der Ansprüche 1 bis 7, **dadurch gekennzeichnet, dass** der Einstelleingriff der Spielreduziereinrichtung (15, 17) mit entweder dem Antriebsglied (20) oder dem Abtriebsglied von einer zumindest im Wesentlichen axial weisenden Führungsbahn (29) und einem entlang der Führungsbahn (29) geführten Eingriffglied (16) gebildet wird.

13. Vorrichtung nach einem der vorhergehenden Ansprüche, **dadurch gekennzeichnet, dass** die Spielreduziereinrichtung (15, 17) ein Einstellglied (15) und ein Rückstellelement (17) umfasst, und dass das Einstellglied (15) über das Abtriebsglied (10) an dem Antriebsglied (20) axial abgestützt ist, wobei das Rückstellelement (17) das Einstellglied (15) axial elastisch in die Einstellposition spannt.

14. Vorrichtung nach einem der vorhergehenden Ansprüche, **dadurch gekennzeichnet, dass** die Antriebsbewegung des Antriebsglieds (20) eine Rotationsbewegung um eine Rotationsachse (R) ist oder zumindest umfasst.

15. Vorrichtung nach einem der vorhergehenden Ansprüche, **dadurch gekennzeichnet,**
**dass** das Abtriebsglied (10) für die Förderung des Produkts eine Translationsbewegung entlang einer Translationsachse (T) ausführt, und das Antriebsglied (20) für die Förderung des Produkts eine Rotationsbewegung um die Translationsachse (T) ausführt,
**dass** die Vorrichtung eine Drehlagerung aufweist, die einen Lagerkörper (30) umfasst, der das Antriebsglied (20) um die Rotationsachse (T) drehbar lagert, wobei der Lagerkörper (30) von dem Gehäuse (1, 2) gebildet werden kann oder vorzugsweise ein separater Lagerkörper (30) ist,
**dass** die Drehlagerung eine erste Stützfläche (31), eine zweite Stützfläche (32), eine dritte Stützfläche (23) und eine vierte Stützfläche (24) aufweist, um das Antriebsglied (20) axial zu sichern,
wobei die erste Stützfläche (31) und die zweite Stützfläche (32) mit dem Lagerkörper (30) axial steif verbunden und entweder voneinander axial abgewandt oder einander axial zugewandt sind, und wobei die dritte Stützfläche (23) der ersten Stützfläche (31) und die vierte Stützfläche (24) der zweiten Stützfläche (32) axial zugewandt und mit dem Antriebsglied (20) axial steif verbunden sind,
und **dass** die Vorrichtung eine weitere Spielreduziereinrichtung (35; 40, 50) umfasst,
wobei die weiter Spielreduziereinrichtung (35; 40, 50) wenigstens zwei der Stützflächen (31, 32, 23, 24), die einander axial zugewandt sind, mit einer Presskraft axial aufeinander zu spannt und **dadurch** ein Axialspiel der Drehlagerung eliminiert oder reduziert.

16. Vorrichtung nach dem vorhergehenden Anspruch, **dadurch gekennzeichnet, dass** die weitere Spielreduziereinrichtung (40, 50) eine die Presskraft erzeugende Feder (50) umfasst, die vorzugsweise wie eine Blattfeder wirkt, vorzugsweise um das Rotationsglied (20) umlaufend gewellt ist.

17. Vorrichtung nach Anspruch 15, **dadurch gekennzeichnet, dass** die weitere Spielreduziereinrichtung (35) die erste Stützfläche oder die zweite Stützfläche (32) bildend mit dem Lagerkörper (30) in einem Einstelleingriff ist, in dem die erste Stützfläche (31) und die zweite Stützfläche (32) relativ zueinander in solch eine Einstellposition verstellt und in der Einstellposition relativ zueinander axial fixiert sind, dass das Axialspiel der Drehlagerung reduziert wird,
und dass die weitere Spielreduziereinrichtung die dritte Stützfläche oder die vierte Stützfläche bildend mit dem Antriebsglied in einem Einstelleingriff ist, in dem die dritte Stützfläche und die vierte Stützfläche relativ zueinander in solch eine Einstellposition verstellt und in der Einstellposition relativ zueinander so gesichert sind, dass das Axialspiel der Drehlagerung reduziert wird.

18. Vorrichtung nach einem der vorhergehenden Ansprüche, **dadurch gekennzeichnet, dass** die Vorrichtung eine Infusionsvorrichtung ist und einen Motor (18) umfasst, der das Antriebsglied (20) antreibt, vorzugsweise drehantreibt.

19. Vorrichtung nach einem der Ansprüche 1 bis 17, **dadurch gekennzeichnet, dass** die Vorrichtung eine Injektionsvorrichtung ist und dass das Antriebsglied um eine Rotationsachse rotatorisch und entlang der Rotationsachse translatorisch relativ zu dem Gehäuse bewegbar gelagert ist und dass das Antriebsglied und das Abtriebsglied miteinander in einem Gewindeeingriff sind.

## Claims

1. A device for administering a fluid product in doses, said device comprising:
a) a housing (1, 2);
b) a reservoir (12) for the product, which is accommodated or formed by the housing (1, 2);
c) a drive member (20) which performs a drive movement and comprises at least one engagement flank (21f);
d) an output member (10) which performs an axial output movement in order to deliver product from the reservoir (12), and comprises at least one engagement flank (11f) which is a flank engagement with the engagement flank (21f) of the drive member (20), such that the drive movement of the drive member (20) generates the output movement of the output member (10);
e) and a play reducing means (25; 15, 17) which, in an adjustment engagement with the drive member (20) and the output member (10), is adjusted relative to the drive member (20) and the output member (10) into an adjustment position and secured in the adjustment position such that an axial play in the flank engagement is reduced;
**characterised in that**
f) the play reducing means (25; 15, 17) is a separate means to the drive member (20) and the output member (10) and comprises at least one adjustment member (25; 15).

2. The device according to claim 1, **characterised in that** the output member (10) comprises other engagement flanks (11f) which successively come into flank engagement with the at least one engagement flank (21f) of the drive member (20), for delivery, and **in that** the play reducing means (25; 15, 17) comprises at least one engagement flank (25f) which is in a flank engagement with one of the other engagement flanks (11f) of the output member (10), said flank engagement forming the adjustment engagement with the output member (10).

3. The device according to any one of the preceding claims, **characterised in that** the flank engagement between the drive member (20) and the output member (10) is a threaded engagement.

4. The device according to any one of the preceding claims, **characterised in that** the adjustment engagement between the play reducing means (25; 15, 17) and the output member (10) is a threaded engagement.

5. The device according to a combination of the preceding two claims, **characterised in that** the output member (10) comprises a thread (11) with which it forms the threaded engagement with the drive member (20) and the threaded engagement with the play reducing means (25; 15, 17).

6. The device according to any one of the preceding claims, **characterised in that** the output member (10) is a threaded rod.

7. The device according to any one of the preceding claims, **characterised in that** the play reducing means (25; 15, 17) is or comprises an inherently axially rigid adjustment member (25; 15) which is in the adjustment engagement with the drive member (20) and in the adjustment engagement with the output member (10).

8. The device according to any one of the preceding claims, **characterised in that** the adjustment engagement between the drive member (20) and the play reducing means (25) is a threaded engagement.

9. The device according to a combination of the preceding two claims, **characterised in that** the play reducing means (25) comprises an inner thread and an outer thread and is in the adjustment engagement with the output member (10) via one of the threads and is in the adjustment engagement with the drive member (20) via the other of the threads.

10. The device according to any one of the preceding claims, **characterised in that** the play reducing means (25) is secured in the adjustment position in a material fit.

11. The device according to the preceding claim, **characterised in that** the play reducing means (25) is secured on the drive member (20) in a material fit.

12. The device according to any one of claims 1 to 7, **characterised in that** the adjustment engagement between the play reducing means (15, 17) and either the drive member (20) or the output member is formed by an at least substantially axially orientated guide track (29) and an engagement member (16) which is guided along the guide track (29).

13. The device according to any one of the preceding claims, **characterised in that** the play reducing means (15, 17) comprises an adjustment member (15) and a restoring element (17), and **in that** the adjustment member (15) is axially supported on the drive member (20) via the output member (10), wherein the restoring element (17) elastically tensions the adjustment member (15) axially into the adjustment position.

14. The device according to any one of the preceding claims, **characterised in that** the drive movement of the drive member (20) is or at least comprises a rotational movement about a rotational axis (R).

15. The device according to any one of the preceding claims, **characterised in that:**
the output member (10) performs a translational movement along a translational axis (T) in order to deliver the product, and the drive member (20) performs a rotational movement about the translational axis (T) in order to deliver the product;
the device comprises a rotary bearing which comprises a bearing body (30) which supports the drive member (20) such that it can rotate about the rotational axis (T), wherein the bearing body (30) can be formed by the housing (1, 2) or is preferably a separate bearing body (30);
the rotary bearing comprises a first support surface (31), a second support surface (32), a third support surface (23) and a fourth support surface (24) in order to axially secure the drive member (20), wherein the first support surface (31) and the second support surface (32) are connected, axially rigid, to the bearing body (30) and either face axially away from each other or face axially towards each other, and wherein the third support surface (23) axially faces the first support surface (31) and the fourth support surface (24) axially faces the second support surface (32) and are connected, axially rigid, to the drive member (20);
the device comprises another play reducing means (35; 40, 50), wherein the other play reducing means (35; 40, 50) axially tensions at least two of the support surfaces (31, 32, 23, 24), which axially face each other, towards each other with a pressing force and thus eliminates or reduces an axial play in the rotary bearing.

16. The device according to the preceding claim, **characterised in that** the other play reducing means (40, 50) comprises a spring (50) which generates the pressing force and preferably acts as a leaf spring and is preferably circumferentially undulated about the rotational member (20).

17. The device according to claim 15, **characterised in that** the other play reducing means (35) forming the first support surface or the second support surface (32) is in an adjustment engagement with the bearing body (30), in which the first support surface (31) and the second support surface (32) are adjusted relative to each other into an adjustment position and axially fixed relative to each other in the adjustment position such that the axial play in the rotary bearing is reduced,
and **in that** the other play reducing means forming the third support surface or the fourth support surface is in an adjustment engagement with the drive member, in which the third support surface and the fourth support surface are adjusted relative to each other into an adjustment position and axially secured relative to each other in the adjustment position such that the axial play in the rotary bearing is reduced.

18. The device according to any one of the preceding claims, **characterised in that** the device is an infusion device and comprises a motor (18) which drives, preferably rotary drives, the drive member (20).

19. The device according to any one of claims 1 to 17, **characterised in that** the device is an injection device, and **in that** the drive member is mounted such that it can be moved rotationally about a rotational axis and translationally along the rotational axis, relative to the housing, and **in that** the drive member and the output member are in a threaded engagement with each other.

## Revendications

1. Dispositif pour administrer de manière dosée un produit fluide, le dispositif comprenant :
a) un boîtier (1, 2),
b) un réservoir (12) reçu ou formé par le boîtier (1, 2) pour le produit,
c) un organe d'entraînement (20) qui exécute un mouvement d'entraînement et présente au moins un flanc d'engrènement (21f),
d) un organe mené (10) qui exécute un mouvement axial mené afin d'extraire le produit du réservoir (12) et qui présente au moins un flanc d'engrènement (11f) qui est en engrènement des flancs (21f) de l'organe d'entraînement (20) de manière que le mouvement d'entraînement de l'organe d'entraînement (20) provoque le mouvement mené de l'organe mené (10),
e) ainsi qu'un dispositif de réduction de jeu (25 ; 15, 17) qui, dans un engrènement de réglage avec l'organe d'entraînement (20) et l'organe mené (10), est déplacé par rapport à l'organe d'entraînement (20) et à l'organe mené (20) dans une position de réglage, et est bloqué dans la position de réglage de manière qu'un jeu axial de l'engrènement des flancs soit réduit,
**caractérisé en ce que**
f) le dispositif de réduction de jeu (25 ; 15, 17) est un dispositif séparé de l'organe d'entraînement (20) et de l'organe mené (10), qui comporte au moins un organe de réglage (25 ; 15).

2. Dispositif selon la revendication 1, **caractérisé en ce que** l'organe mené (10) présente d'autres flancs d'engrènement (11f) qui, pour le transport, viennent successivement en engrènement des flancs avec le au moins un flanc d'engrènement (21f) de l'organe d'entraînement (20), et **en ce que** le dispositif de réduction de jeu (25 ; 15, 17) présente au moins un flanc d'engrènement (25f) qui est en engrènement des flancs avec l'un des autres flancs d'engrènement (11f) de l'organe mené (10), formant l'engrènement de réglage avec l'organe mené (10).

3. Dispositif selon l'une quelconque des revendications 1 ou 2, **caractérisé en ce que** l'engrènement des flancs de l'organe d'entraînement (20) avec l'organe mené (10) est un engrènement fileté.

4. Dispositif selon l'une quelconque des revendications 1 à 3, **caractérisé en ce que** l'engrènement de réglage du dispositif de réduction de jeu (25 ; 15, 17) avec l'organe mené (10) est un engrènement fileté.

5. Dispositif selon une combinaison des deux revendications 3 et 4 précédentes, **caractérisé en ce que** l'organe mené (10) présente un filetage (11) avec lequel il forme l'engrènement fileté avec l'organe d'entraînement (20) et l'engrènement fileté avec le dispositif de réduction de jeu (25 ; 15, 17).

6. Dispositif selon l'une quelconque des revendications 1 à 5, **caractérisé en ce que** l'organe mené (10) est une tige filetée.

7. Dispositif selon l'une quelconque des revendications 1 à 6, **caractérisé en ce que** le dispositif de réduction de jeu (25 ; 15, 17) est ou comporte un organe de réglage (25 ; 15) axialement rigide qui est en engrènement de réglage avec l'organe d'entraînement (20) et en engrènement de réglage avec l'organe mené (10).

8. Dispositif selon l'une quelconque des revendications 1 à 7, **caractérisé en ce que** l'engrènement de réglage entre l'organe d'entraînement (20) et le dispositif de réduction de jeu (25) est un engrènement fileté.

9. Dispositif selon une combinaison des deux revendications 7 et 8 précédentes, **caractérisé en ce que** le dispositif de réduction de jeu (25) présente un filetage intérieur et un filetage extérieur et, avec l'un des filetages, il est en prise de réglage avec l'organe mené (10) et, avec l'autre filetage, il est en prise de réglage avec l'organe d'entraînement (20).

10. Dispositif selon l'une quelconque des revendications 1 à 9, **caractérisé en ce que** le dispositif de réduction de jeu (25) est bloqué en position de réglage par adhérence de matière.

11. Dispositif selon l'une quelconque des revendications 1 à 10, **caractérisé en ce que** le dispositif de réduction de jeu (25) est bloqué sur l'organe d'entraînement (20) par adhérence de matière.

12. Dispositif selon l'une quelconque des revendications 1 à 7, **caractérisé en ce que** l'engrènement de réglage du dispositif de réduction de jeu (15, 17) est formé, soit avec l'organe d'entraînement (20), soit avec l'organe mené, par une voie de guidage (29) dirigée au moins sensiblement axialement et par un organe d'engrènement (16) guidé le long de la voie de guidage (29).

13. Dispositif selon l'une quelconque des revendications 1 à 12, **caractérisé en ce que** le dispositif de réduction de jeu (15, 17) comprend un organe de réglage (15) et un élément de rappel (17), et **en ce que** l'organe de réglage (15) est soutenu axialement sur l'organe d'entraînement (20), à travers l'organe mené (10), l'élément de rappel (17) tendant axialement et élastiquement l'organe de réglage (15) dans la position de réglage.

14. Dispositif selon l'une quelconque des revendications 1 à 13, **caractérisé en ce que** le mouvement d'entraînement de l'organe d'entraînement (20) est un mouvement de rotation autour d'un axe de rotation (R) ou comprend au moins celui-ci.

15. Dispositif selon l'une quelconque des revendications 1 à 14, **caractérisé en ce que**
l'organe mené (10) exécute pour le transport du produit un mouvement de translation le long d'un axe de translation (T), et l'organe d'entraînement (20) exécute pour le transport du produit un mouvement de rotation autour de l'axe de translation (T), **en ce que** le dispositif comporte un support en rotation qui comprend un corps de palier (30) qui supporte l'organe d'entraînement (20) de manière qu'il puisse tourner autour de l'axe de rotation (T), le corps de palier (30) pouvant être formé par le boîtier (1, 2) ou étant de préférence un corps de palier (30) séparé,
**en ce que** le support en rotation présente une première surface d'appui (31), une deuxième surface d'appui (32), une troisième surface d'appui (23) et une quatrième surface d'appui (24) afin de bloquer axialement l'organe d'entraînement (20),
la première surface d'appui (31) et la seconde surface d'appui (32) étant reliées axialement rigidement au corps de palier (30) et étant soit tournées axialement à l'opposé l'une de l'autre, soit tournées axialement l'une vers l'autre, et la troisième surface d'appui (23) étant tournée axialement vers la première surface d'appui (31) et la quatrième surface d'appui (24) étant tournée axialement vers la deuxième surface d'appui (32) et étant reliée axialement rigidement à l'organe d'entraînement (20),
et **en ce que** le dispositif comprend un autre dispositif de réduction de jeu (35 ; 40, 50), l'autre dispositif de réduction de jeu (35 ; 40, 50) serrant axialement l'une sur l'autre au moins deux des surfaces d'appui (31, 32, 23, 24) qui sont tournées axialement l'une vers l'autre, avec une force de pression, et éliminant ou réduisant ainsi un jeu axial du support en rotation.

16. Dispositif selon la revendication 15, **caractérisé en ce que** l'autre dispositif de réduction de jeu (40, 50) comprend un ressort (50) produisant la force de pression qui agit de préférence comme un ressort à lame, et qui est de préférence ondulé tout autour de l'organe de rotation (20).

17. Dispositif selon la revendication 15, **caractérisé en ce que** l'autre dispositif de réduction de jeu (35) formant la première surface d'appui ou la deuxième surface d'appui (32), est en engrènement de réglage avec le corps de palier (30), engrènement dans lequel la première surface d'appui (31) et la deuxième surface d'appui (32) sont déplacées l'une par rapport à l'autre dans une position de réglage et sont fixées axialement l'une par rapport à l'autre dans la position de réglage, de manière que le jeu axial du support en rotation soit réduit, et **en ce que** l'autre dispositif de réduction de jeu, formant la troisième surface d'appui ou la quatrième surface d'appui, est en engrènement de réglage avec l'organe d'entraînement, engrènement dans lequel la troisième surface d'appui et la quatrième surface d'appui sont déplacées l'une par rapport à l'autre dans une position de réglage et sont bloquées l'une par rapport à l'autre dans la position de réglage de manière que le jeu axial du support en rotation soit réduit.

18. Dispositif selon l'une quelconque des revendications 1 à 17, **caractérisé en ce que** le dispositif est un dispositif de perfusion et comprend un moteur (18) qui entraîne l'organe d'entraînement (20), de préférence en rotation.

19. Dispositif selon l'une quelconque des revendications 1 à 17, **caractérisé en ce que** le dispositif est un dispositif d'injection et **en ce que** l'organe d'entraînement est supporté mobile en rotation autour d'un axe de rotation et en translation le long de l'axe de rotation, par rapport au boîtier, et **en ce que** l'organe d'entraînement et l'organe mené sont en engrènement fileté l'un avec l'autre.
